# EUROPEAN PATENT SPECIFICATION

(11) **EP 4 233 955 B1**
(45) Date of publication and mention of the grant of the patent: **12.08.2026**
(21) Application number: 23163927.9
(22) Date of filing: 30.05.2018
(51) Int. Cl.: A61M 16/06

(54) **HEADGEAR FOR A PATIENT INTERFACE**
KOPFBEDECKUNG FÜR EINE PATIENTENSCHNITTSTELLE
CASQUE POUR INTERFACE PATIENT

(30) Priority: 30.05.2017 US 201762512587 P
(43) Date of publication of application: 30.08.2023
(62) Document number(s) of the earlier application(s) in accordance with Art. 76 EPC: 18808781.1 / 3 630 249
(73) Proprietor: Fisher & Paykel Healthcare Limited, East Tamaki Auckland 2013 (NZ)
(72) Inventor: WHITE, Craig Karl, 2013 Auckland (NZ); PROLE, Dylan Peter, 2013 Auckland (NZ); HOLYOAKE, Bruce Gordon, 2013 Auckland (NZ); OSBORNE, Hamish Adrian, 2013 Auckland (NZ); FLYNN, Cormac Nicholas, 2013 Auckland (NZ)
(74) Representative: Forresters IP LLP

(56) References cited:
- WO-A1-2012/045127
- WO-A1-2012/140514
- WO-A1-2017/031411
- US-A- 4 774 946
- US-A1- 2007 240 721
- US-A1- 2012 285 463
- US-A1- 2016 256 655
- US-B2- 7 735 490

## Description

### TECHNICAL FIELD

The present disclosure generally relates to a headgear for a patient interface such as a face mask, nasal mask or nasal cannula.

### BACKGROUND

Patients may lose respiratory function during anaesthesia, or sedation, or more generally during medical procedures. Prior to a medical procedure a patient may be pre-oxygenated by a medical professional to provide a reservoir of oxygen saturation, and this pre-oxygenation and CO2 flushing/washout may be carried out with a high flow therapy via a nasal cannula or other patient interface.

A patient interface may be held in place on a patient's face by a headgear. The headgear has head straps to extend around the head of a patient or user, to position and hold the patient interface in the correct position to provide respiratory gases to the patient. During fitting or removal of a nasal cannula or patient interface from the conscious or sedated patient it may be necessary for the medical professional to manipulate the position of the patient's head in order to apply or remove a head strap, or the like. As a result, this may cause some disturbance to the patient's position and/or comfort, provide awkward or uncomfortable mechanical loading on the medical professional, disturb the patient's hairnet and/or increase the time of the medical procedure.

Additionally, elastic and/or size-adjustable head straps may present disadvantages. It may be difficult for the medical professional to grip or adjust a head strap - particularly with gloved hands. When attempting to grip an elastic head strap the medical professional may attempt to slide a finger between the head strap and patient's head, or try to pull the head strap away from the patient's head by pinching the head strap, or drag a head strap across a patient's hair and skin. As a result, the medical professional may inadvertently dislodge the patient's hairnet or pull the patient's hair or skin, or cause discomfort.

US 2016/256655 A1 discloses a patient interface having a positioning and stabilising structure. The positioning and stabilising structure may comprise at least one strap having elastic properties, and at least one rigidiser arm. US 7735490 B2 discloses an adjustable nasal cannula apparatus. WO 2012/140514 A1 discloses a flexible headgear assembly.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

### SUMMARY OF THE DISCLOSURE

It is an object of embodiments disclosed herein to provide an improved headgear which will go at least some way towards addressing one or more of the above mentioned disadvantages or which will at least provide the industry or public with a useful choice.

When applying and removing a patient interface to and from a patient, it is desirable to avoid movement of the patient's head, disruption of the patient's hairnet, and improve handling and positioning of a patient interface.

The present invention provides a headgear for a patient interface according to claim 1, and a patient interface assembly according to claim 15.

A headgear disclosed herein comprises:
at least one headgear member, and
a pair of flexible joints adapted to connect a said at least one headgear member to a patient interface and/or a pair of flexible joints connecting one of the headgear members to an adjacent headgear member,
each flexible joint allowing free relative movement between the at least one headgear member and the patient interface or between the adjacent headgear members.

In accordance with an aspect of the disclosure, there is provided, a headgear comprising:
at least one headgear member, and
a pair of flexible joints adapted to connect a said headgear member or headgear members to a patient interface and/or a pair of flexible joints connecting a said headgear member to adjacent headgear members,
each flexible joint allowing free relative movement between the headgear member or members and the patient interface or between the headgear member and adjacent headgear members.

The following additional features or embodiments are provided:
In some embodiments the flexible joint allows free relative movement between the headgear member or members and the patient interface or between the headgear member and adjacent headgear members with at least two degrees of freedom.

In some embodiments the flexible joint allows free relative movement between the headgear member or members and the patient interface or between the headgear member and adjacent headgear members with at least one degree of freedom.

In some embodiments the flexible joint allows for elongation (optionally lengthwise or in a direction along the at least one headgear member) to allow free relative movement between the headgear member or members and the patient interface or between the headgear member and adjacent headgear members.

In some embodiments, each flexible joint comprises a hinge, optionally the hinge is a living hinge.

In some embodiments, the flexible joint is adapted to bend or fold laterally to a longitudinal axis of an end portion of a said headgear member.

In some embodiments, the flexible joint is or comprises a unitary member.

In some embodiments, the flexible joint is a member formed from a soft flexible material.

In some embodiments, the flexible joint is an elastomeric member.

In some embodiments, the flexible joint is a hollow or tubular member.

In some embodiments, the flexible joint, or the at least one headgear member, comprises a connecting portion or connection arrangement.

In some embodiments, the connecting portion or connection arrangement is configured to provide for connection with one or more of: an interface (or a part of an interface), an arm adapted to connect to or form part of a patient interface.

In some embodiments, the connecting portion or connection arrangement is configured to provide for a pivotal connection.

In some embodiments, the connecting portion or connection arrangement comprises a recess, or aperture configured to receive a projection or boss, optionally the projection or boss is located on an interface or part of an interface, optionally the recess or aperture is located on the flexible joint.

In some embodiments, the connecting portion or connection arrangement comprises a projection or boss configured to be received by a recess or aperture, optionally the projection or boss is located on the flexible joint, optionally the recess or aperture is located on an interface or part of an interface.

In some embodiments, the at least one headgear member is integral with the flexible joint.

In some embodiments the headgear and an interface, or a part of an interface, or an arm adapted to connect to or form part of an interface may be integrally formed.

In some embodiments, the flexible joint elastically deforms (without permanent deformation) under use conditions.

In some embodiments, the flexible joint allows for rotation of a said headgear member relative to an adjacent headgear member about a longitudinal axis of an end portion of the headgear member attached to the adjacent headgear member.

In some embodiments, a said headgear member is moveably attached to the flexible joint to move relative to an adjacent headgear member along a longitudinal axis of the headgear member.

In some embodiments, the headgear member is telescopically attached to the flexible joint.

In some embodiments, the headgear member is telescopically received in an end portion of the flexible joint.

In some embodiments, the flexible joint comprises at least one passageway (optionally the flexible joint is or comprises a hollow or tubular member), the at least one passageway configured to allow for the passage of the head gear member.

In some embodiments, the headgear member is configured to move or slide within the at least one passageway

In some embodiments, the headgear member comprises at least one stop to limit movement of the headgear member relative to the flexible joint and/or the at least one passageway, optionally, the at least one stop is located at an end of the headgear member.

In some embodiments, the at least one stop comprises one or more of:
i. a protrusion
ii. a recess
iii. a barbed end
iv. a localised increase or decrease in thickness, or cross-sectional area of the headgear member, optionally, relative to the size of the at least one passageway.

In some embodiments, the headgear member comprises one or more of a first stop and/or a second stop, wherein the first stop to provide for a first limit of movement of the head gear member relative to the flexible joint and/or the at least one passageway, and wherein the second stop to provide for a second limit of movement of the head gear member relative to the flexible joint and/or the at least one passageway.

In some embodiments, the at least one headgear member is/are releasably attachable, or connectable and detachable or disconnectable, with or from an adjacent headgear member and/or the flexible joint.

In some embodiments, an end of the at least one headgear member extends past or beyond the flexible joint.

In some embodiments, the headgear comprises at least one pair of said flexible joints, and each one of the pair of flexible joints engages a respective side of the user's head in use.

In some embodiments, the modulus of elasticity of the at least one of the headgear members is at least about 0.5 GPa, or at least about 1GPa, or at least about 1.5GPa, or at least about 1.8GPa, or at least about 2GPa, or at least about 3 GPa.

In some embodiments, the modulus of elasticity of the at least one of the headgear members is at least about 1GPa or at least about 1.5GPa, or at least about 1.8GPa, or at least about 2GPa.

In some embodiments, the flexible joint is formed from a material with a modulus of elasticity of less than about 0.2GPa, or less than about 0.1GPa, or less than about 0.06GPa.

In some embodiments, at least one of the headgear members is formed from a stiff resilient material and the flexible joint is formed from a soft flexible material, wherein the modulus of elasticity of the stiff resilient material is many times greater than the modulus of elasticity of the soft flexible material.

In some embodiments, the modulus of elasticity of the stiff resilient material is at least 10 times the modulus of elasticity of the soft flexible material, or at least 10, or 20, or 40, or 100, or 200 times the modulus of elasticity of the soft flexible material.

The headgear comprises a pair of positioning tabs or positioning members connected to, or forming part of, at least one said headgear member.

In some embodiments, the at least one positioning tab or positioning member allowing a user to apply and remove said headgear.

In some embodiments, the positioning tab or positioning member is provided by or comprises a substantially u-shaped portion.

In some embodiments, the positioning tab or positioning member comprises:
a first portion extending outward from a headgear member in use (e.g. outwardly from the user's head),
a second portion extending toward a headgear member in use (e.g. toward from the user's head),
optionally, the positioning tab or positioning member comprises an intermediate portion located intermediate the first portion and the second portion, the intermediate portion extending substantially parallel to a headgear member in use (e.g. parallel to the user's head).

In some embodiments, the at least one positioning tab or positioning member is integrally formed with said headgear member.

In some embodiments, the headgear comprises a pair of engagement portions, each engagement portion configured to engage a side of the user's head in use.

In some embodiments, the pair of flexible joints provide or comprise the engagement portions.

In some embodiments, the pair of flexible joints and/or the at least one headgear member provide or comprise the engagement portions.

In some embodiments, the engagement portions comprise a textured surface, optionally the textured surface comprises one or more of: a knurled portion, a waved surface, a ribbed surface, a roughened surface, or may comprise micro or nano projections.

In some embodiments, the headgear comprises:
a resilient headband shaped to fit a user's head with each end of the headband located to a respective side of the user's head,
a pair of arms, each arm attached to the resilient headband, each arm adapted to connect to or part of a patient interface, and
wherein each arm is attached to the headband by a said flexible joint, the flexible joint allowing the arm to move freely relative to the headband with at least two degrees of freedom.

In some embodiments, each arm is attached to an end or end portion of the headband.

In some embodiments, each arm is a resilient arm.

The resilient headband is shaped to fit a user's head with each end of the headband located to and biased against a respective side of the user's head.

In an un-deflected condition the distance between the ends of the headband is less than a width of a user's head so that defection of the resilient headband to fit the user's head biases the ends of the resilient headband against sides of the user's head.

In some embodiments, headband is shaped to fit over the top of a user's head.

In some embodiments, the headband comprises a central portion and an end portion(s), the end portion(s) located to a side or a respective side of the user's head, and wherein the central portion is angled with respect to the end portion(s), optionally, to fit about a rear portion of the user's head in use.

In some embodiments, the headband has a central portion, and two end portions, wherein the central portion is relatively stiffer or more stiff or less flexible, than the end portions.

In some embodiments, the central portion has a larger cross-sectional area than the end portions.

In some embodiments, the central portion is made of a first material and the end portions are made of a second material, wherein the first material is relatively stiffer or more stiff or less flexible, than the second material.

In some embodiments, the headgear comprises a second headband.

In some embodiments, the second headband comprises an elastic strap or band, optionally the elastic strap or band is elastic, and/or stretchable and/or elongatable.

In some embodiments, the second headband is engageable with a rear part of a user's head, optionally the second headband is configured to engage with a portion of the user's head rearward of a crown of a user's head.

In some embodiments, the second headband is adapted to be connected to the headband, optionally the headband comprises a connection feature adapted to allow for connection and disconnection of the headband and the second headband, optionally the connection feature comprises a groove, clip or buckle.

In some embodiments, the headband is shaped so that the flexible joints connecting the arms to the headband are located in use:
i) in proximity to the temporal bone of the user so that the flexible joints occupy an inward contour in front of the user's ear and above the zygomatic bone or arch when viewed in side profile, or
ii) forward of the ear in proximity to the zygomatic bone when viewed in side profile.

In some embodiments, in use, the headgear has a first lateral distance between the flexible joints, a second lateral distance between left and right side portions of the headband intermediate the flexible joints and a sagittal plane position of the headband, and a third lateral distance between ends or end portions of the arms attached to or attachable to the patient interface,
wherein each lateral distance is across and perpendicular to the sagittal plane, and
wherein the first lateral distance is less than the second and third lateral distances.

In some embodiments, the headgear has a first lateral distance between the flexible joints, a second lateral distance between left and right side portions of the headband intermediate the flexible joints and a sagittal plane position of the headband, and a third lateral distance between ends or end portions of the arms attached to or attachable to the patient interface,
wherein each lateral distance is across and perpendicular to the sagittal plane, and
wherein the first lateral distance is less than the second and third lateral distances.

In some embodiments, the second lateral distance is greater than the third lateral distance.

In some embodiments, the arms are integrally formed with the patient interface.

In some embodiments, the patient interface and the headgear form a continuous loop comprising an upper portion and a lower portion joined by the pair of flexible joints.

In some embodiments, the flexible joint, a or the positioning tab, or the at least one headgear members comprises at least one channel, optionally the channel extending from a front side of the flexible joint, or the at least one headgear member to a rear side of the flexible joint, or the at least one headgear member, or the or an engagement portion.

In accordance with an aspect of the disclosure, there is provided, a headgear for a patient interface comprising:
a resilient headband shaped to fit a user's head with each end of the headband located to a respective side of the user's head,
a pair of resilient arms, each arm attached to the resilient headband, each arm adapted to connect to or form part of a patient interface, and
wherein each arm is attached to the headband by a flexible joint, the flexible joint allowing the arm to move freely relative to the headband with at least two degrees of freedom.

In some embodiments, each flexible joint comprises a hinge, optionally the hinge is a living hinge.

In some embodiments, each arm is attached to an end or end portion of the headband.

In some embodiments, the flexible joint is adapted to bend laterally to a longitudinal axis of an end portion of the headband.

In some embodiments, the flexible joint is or comprises a unitary member.

In some embodiments, the flexible joint is a member formed from a soft flexible material.

In some embodiments, the flexible joint is an elastomeric member.

In some embodiments, the flexible joint is a hollow or tubular member.

In some embodiments, the flexible joint, or the at least one headgear member, comprises a connecting portion or connection arrangement.

In some embodiments, the connecting portion or connection arrangement is configured to provide for connection with one or more of: an interface (or a part of an interface), an arm adapted to connect to or form part of a patient interface.

In some embodiments, the connecting portion or connection arrangement is configured to provide for a pivotal connection.

In some embodiments, the connecting portion or connection arrangement comprises a recess, or aperture configured to receive a projection or boss, optionally the projection or boss is located on an interface or part of an interface, optionally the recess or aperture is located on the flexible joint.

In some embodiments, the connecting portion or connection arrangement comprises a projection or boss configured to be received by a recess or aperture, optionally the projection or boss is located on the flexible joint, optionally the recess or aperture is located on an interface or part of an interface.

In some embodiments, the at least one headgear member is integral with the flexible joint.

In some embodiments the headgear and an interface, or a part of an interface, or an arm adapted to connect to or form part of an interface may be integrally formed.

In some embodiments, the flexible joint elastically deforms (without permanent deformation) in use to allow the arm to move freely relative to the headband.

In some embodiments, the flexible joint allows for rotation of the arm relative to the headband about a longitudinal axis of an end portion of the arm attached to the headband.

In some embodiments, the arm is moveably attached to the flexible joint to move relative to the headband along a longitudinal axis of the arm.

In some embodiments, the arm is telescopically attached to the flexible joint.

In some embodiments, the arm is telescopically received in an end portion of the flexible joint.

In some embodiments, the flexible joint comprises at least one passageway (optionally the flexible joint is or comprises a hollow or tubular member), the at least one passageway configured to allow for the passage of the head gear member.

In some embodiments, the headgear member is configured to move or slide within the at least one passageway

In some embodiments, the headgear member comprises at least one stop to limit movement of the headgear member relative to the flexible joint and/or the at least one passageway, optionally, the at least one stop is located at an end of the headgear member.

In some embodiments, the at least one stop comprises one or more of:
i. a protrusion
ii. a recess
iii. a barbed end
iv. a localised increase or decrease in thickness, or cross-sectional area of the headgear member, optionally, relative to the size of the at least one passageway.

In some embodiments, the headgear member comprises one or more of a first stop and/or a second stop, wherein the first stop to provide for a first limit of movement of the head gear member relative to the flexible joint and/or the at least one passageway, and wherein the second stop to provide for a second limit of movement of the head gear member relative to the flexible joint and/or the at least one passageway.

In some embodiments, the at least one headgear member is/are releasably attachable, or connectable and detachable or disconnectable, with or from an adjacent headgear member and/or the flexible joint.

In some embodiments, an end of the at least one headgear member extends past or beyond the flexible joint.

In some embodiments, the flexible joint at or adjacent each end of the headband engages a respective side of the user's head in use.

In some embodiments, the modulus of elasticity of the at least one of the headgear members is at least about 0.5 GPa, or at least about 1GPa, or at least about 1.5GPa, or at least about 1.8GPa, or at least about 2GPa, or at least about 3 GPa.

In some embodiments, the modulus of elasticity of the headband is at least about 1GPa or at least about 1.5GPa, or at least about 1.8GPa, or at least about 2GPa.

In some embodiments, the modulus of elasticity of the arms is at least about 1GPa or at least about 1.5GPa, or at least about 1.8GPa, or at least about 2GPa.

In some embodiments, the flexible joint is formed from a material with a modulus of elasticity of less than about 0.2GPa, or less than about 0.1GPa, or less than about 0.06GPa.

In some embodiments, the headband, or the headband and arms, is formed from a stiff resilient material and the flexible joint is formed from a soft flexible material, wherein the modulus of elasticity of the stiff resilient material is many times greater than the modulus of elasticity of the soft flexible material.

In some embodiments, the modulus of elasticity of the stiff resilient material is at least 10 times the modulus of elasticity of the soft flexible material, or at least 20, or 40, or 100, or 200 times the modulus of elasticity of the soft flexible material.

In some embodiments, the headgear comprises at least one positioning tab connected to at least one said headgear member.

In some embodiments, the headgear comprises at least one positioning tab or positioning member connected to, or forming part of, at least one said headgear member.

In some embodiments, the at least one positioning tab or positioning member allowing a user to apply and remove said headgear.

In some embodiments, the positioning tab or positioning member is provided by or comprises a substantially u-shaped portion.

In some embodiments, the positioning tab or positioning member comprises:
a first portion extending outward from a headgear member in use (e.g. outwardly from the user's head),
a second portion extending toward a headgear member in use (e.g. toward from the user's head),
optionally, the positioning tab or positioning member comprises an intermediate portion located intermediate the first portion and the second portion, the intermediate portion extending substantially parallel to a headgear member in use (e.g. parallel to the user's head).

In some embodiments, the at least one positioning tab or positioning member is integrally formed with said headgear member.

In some embodiments, the headgear comprises a pair of engagement portions, each engagement portion configured to engage a side of the user's head in use.

In some embodiments, the pair of flexible joints provide or comprise the engagement portions.

In some embodiments, the pair of flexible joints and/or the at least one headgear member provide or comprise the engagement portions.

In some embodiments, the engagement portions comprise a textured surface, optionally the textured surface comprises one or more of: a knurled portion, a waved surface, a ribbed surface, a roughened surface, or may comprise micro or nano projections optionally said micro projections is/are about 1x10-6 m, optionally said nano projections is/are about 1x10-9 m.

In some embodiments, the resilient headband is shaped to fit a user's head with each end of the headband located to and biased against a respective side of the user's head.

In some embodiments, in an un-deflected condition the distance between the ends of the headband is less than a width of a user's head so that defection of the headband to fit the user's head biases the ends of the headband against sides of the user's head.

In some embodiments, the headband is shaped to fit over the top of a user's head.

In some embodiments, the headband comprises a central portion and an end portion(s), the end portion(s) located to a side or a respective side of the user's head, and wherein the central portion is angled with respect to the end portion(s), optionally, to fit about a rear portion of the user's head in use.

In some embodiments, the headband has a central portion, and two end portions, wherein the central portion is relatively stiffer or more stiff or less flexible, than the end portions.

In some embodiments, the central portion has a larger cross-sectional area than the end portions.

In some embodiments, the central portion is made of a first material and the end portions are made of a second material, wherein the first material is relatively stiffer or more stiff or less flexible, than the second material.

In some embodiments, the headgear comprises a second headband.

In some embodiments, the second headband comprises an elastic strap or band, optionally the elastic strap or band is elastic, and/or stretchable and/or elongatable.

In some embodiments, the second headband is engageable with a rear part of a user's head, optionally the second headband is configured to engage with a portion of the user's head rearward of a crown of a user's head.

In some embodiments, the second headband is adapted to be connected to the headband, optionally the headband comprises a connection feature adapted to allow for connection and disconnection of the headband and the second headband, optionally the connection feature comprises a groove, clip or buckle.

In some embodiments, the headband is shaped so that the flexible joints connecting the arms to the headband are located in use:
i) in proximity to the temporal bone of the user so that the flexible joints occupy an inward contour in front of the user's ear and above the zygomatic bone or arch when viewed in side profile, or
ii) forward of the ear in proximity to the zygomatic bone when viewed in side profile.

In some embodiments, in use, the headgear has a first lateral distance between the flexible joints, a second lateral distance between left and right side portions of the headband intermediate the flexible joints and a sagittal plane position of the headband, and a third lateral distance between ends or end portions of the arms attached to or attachable to the patient interface,
wherein each lateral distance is across and perpendicular to the sagittal plane, and
wherein the first lateral distance is less than the second and third lateral distances.

In some embodiments, the headgear has a first lateral distance between the flexible joints, a second lateral distance between left and right side portions of the headband intermediate the flexible joints and a sagittal plane position of the headband, and a third lateral distance between ends or end portions of the arms attached to or attachable to the patient interface,
wherein each lateral distance is across and perpendicular to the sagittal plane, and
wherein the first lateral distance is less than the second and third lateral distances.

In some embodiments, the second lateral distance is greater than the third lateral distance.

In accordance with an aspect of the disclosure, there is provided, a patient interface assembly comprising
a patient interface,
a headgear, wherein the headgear is the headgear of any one of the preceding first, second and third aspects described above or below, the headgear configured to, in-use, locate the patient interface upon a patient's face.

In accordance with an aspect of the disclosure, there is provided, a headgear for a patient interface comprising:
at least one headgear member, and
at least one positioning member connected to, or forming part of at least one said headgear member.

In some embodiments, the at least one positioning member allowing a user to apply and remove said headgear.

In some embodiments, the positioning member is provided by, or comprises, a substantially u-shaped portion.

In some embodiments, the positioning member comprises:
a first portion extending outward from a headgear member in-use (e.g. outwardly from the user's head),
a second portion extending toward a headgear member in-use (e.g. toward from the user's head),
optionally the positioning member comprises an intermediate portion located intermediate the first portion and the second portion, the intermediate portion extending substantially parallel to a headgear member in use (e.g. parallel to the user's head).

In some embodiments, the at least one positioning member is integrally formed with said headgear member.

In some embodiments, the at least one positioning member is or comprises of a positioning tab.

In some embodiments, the flexible joint is a member formed from a soft flexible material.

In some embodiments, the positioning tab or positioning member is integrally formed with the flexible joint, optionally, the flexible joint and tab is or comprises a unitary member.

In some embodiments, the positioning tab or positioning member is marked with visual indicia to illustrate a direction to pull the tab to remove the headgear from a user's head.

In some embodiments, the positioning tab or positioning member protrudes outward from a headgear member in use (e.g. outwardly from the user's head).

The headgear member is a resilient headband and the headgear comprises a pair of positioning tabs or positioning member, each positioning tab adjacent an end or end portion of the resilient headband to be located at a side of the user's head in use.

In some embodiments, the flexible joint, or the at least one headgear members, and/or the at least one positioning tab or positioning member, and/or the or an engagement portion comprises at least one channel.

In some embodiments, the channel extends from a front side of the flexible joint, or the at least one headgear member, or the at least one positioning tab or positioning member, or the or an engagement portion to a rear side of the flexible joint, or the at least one headgear member, or the at least one positioning tab or positioning member, or the or an engagement portion.

In some embodiments, in use, the channel forms a channel passageway between a surface of the flexible joint, or the at least one headgear members, or the at least one positioning tab, or positioning member, or the or an engagement portion, and a patient's head.

In some embodiments, the channel is provided by a substantially U-shaped portion.

In some embodiments, the headgear comprises:
a resilient headband shaped to fit a user's head with each end of the headband located to a respective side of the user's head,
a pair of arms, each arm attached to the resilient headband, each arm adapted to connect to or part of a patient interface, and
wherein at least one positioning tab is connected to the headband or a said arm.

In some embodiments, each arm is a resilient arm.

In some embodiments, each arm is attached to an end or end portion of the headband.

In some embodiments, the headgear comprises a pair of engagement portions, each engagement portion configured to engage a side of the user's head in use.

In some embodiments, the headband comprises a central portion and an end portion(s), the end portion(s) located to a side or a respective side of the user's head, and wherein the central portion is angled with respect to the end portion(s), optionally in-use to fit about a rear potion of the user's head.

In some embodiments, the headgear comprises:
a resilient headband shaped to fit over the top of a user's head with each end of the headband located and biased to a respective side of the user's head, and
a pair of engagement portions, each engagement portion configured to engage a side of the user's head,
wherein in use the headgear has a first lateral distance between the engagement portions, a second lateral distance between left and right side portions of the headband that are intermediate the engagement portions and a sagittal plane position of the headband, and a third lateral distance between ends or end portions of the headgear attached to or attachable to the patient interface,
   wherein each lateral distance is across and perpendicular to the sagittal plane, and
   wherein the first lateral distance is less than the second and third lateral distances.

In some embodiments, the patient interface and headgear form a continuous loop comprising an upper portion and a lower portion joined by the pair of flexible joints.

In accordance with an aspect of at least one of the embodiments disclosed herein the headgear for a patient interface comprises:
at least one headgear member, and
at least one positioning tab connected to at least one said headgear member.

The headgear comprises a pair of flexible joints adapted to connect a said headgear member to a patient interface, and/or wherein the headgear comprises a pair of flexible joints connecting one of the headgear members to an adjacent headgear member, each flexible joint allowing free relative movement between adjacent headgear members.

In some embodiments, the positioning tab is integrally formed with the flexible joint, for example the flexible joint and tab is or comprises a unitary member.

In some embodiments, the positioning tab is marked with visual indicia to illustrate a direction to pull the tab to remove the headgear from a user's head.

In some embodiments, the positioning tab protrudes outward from a headgear member in use (e.g. outwardly from the user's head).

The headgear member is a resilient headband and the headgear comprises a pair of positioning tabs, each positioning tab adjacent an end or end portion of the resilient headband to be located at a side of the user's head in use.

In some embodiments, the flexible joint, or the at least one headgear members, and/or the at least one positioning tab or positioning member, and/or the or an engagement portion comprises at least one channel.

In some embodiments, the channel extends from a front side of the flexible joint, or the at least one headgear member, or the at least one positioning tab or positioning member, or the or an engagement portion to a rear side of the flexible joint, or the at least one headgear member, or the at least one positioning tab or positioning member, or the or an engagement portion.

In some embodiments, in use, the channel forms a channel passageway between a surface of the flexible joint, or the at least one headgear members, or the at least one positioning tab, or positioning member, or the or an engagement portion, and a patient's head.

In some embodiments, the channel is provided by a substantially U-shaped portion.

In some embodiments, the headgear comprises:
a resilient headband shaped to fit a user's head with each end of the headband located to a respective side of the user's head,
a pair of arms, each arm attached to the resilient headband, each arm adapted to connect to or part of a patient interface, and
wherein at least one positioning tab is connected to the headband or a said arm.

In some embodiments, each arm is a resilient arm.

In some embodiments, each arm is attached to an end or end portion of the headband.

In some embodiments, the headgear comprises a pair of engagement portions, each engagement portion configured to engage a side of the user's head in use.

In some embodiments, the pair of flexible joints provide or comprise the engagement portions.

In some embodiments, the pair of flexible joints and/or the at least one headgear member provide or comprise the engagement portions.

In some embodiments, the engagement portions comprise a textured surface, optionally the textured surface comprises one or more of: a knurled portion, a waved surface, a ribbed surface, a roughened surface, or may comprise micro or nano projections.

In some embodiments, the headband comprises a central portion and an end portion(s), the end portion(s) located to a side or a respective side of the user's head, and wherein the central portion is angled with respect to the end portion(s), optionally in-use to fit about a rear potion of the user's head.

In some embodiments, the headgear comprises:
a resilient headband shaped to fit over the top of a user's head with each end of the headband located and biased to a respective side of the user's head, and
a pair of engagement portions, each engagement portion configured to engage a side of the user's head,
wherein in use the headgear has a first lateral distance between the engagement portions, a second lateral distance between left and right side portions of the headband that are intermediate the engagement portions and a sagittal plane position of the headband, and a third lateral distance between ends or end portions of the headgear attached to or attachable to the patient interface,
wherein each lateral distance is across and perpendicular to the sagittal plane, and
wherein the first lateral distance is less than the second and third lateral distances.

In some embodiments, a patient interface and the headgear form a continuous loop comprising an upper portion and a lower portion joined by the pair of flexible joints.

In accordance with an aspect of the disclosure, there is provided, a headgear the headgear comprising:
at least one headgear member
at least one sliding member, the sliding member being moveable attached to the headband and configured to slide relative to the headband.

In some embodiments, there are a pair of sliding members, optionally located on each side of a headband.

In some embodiments, the at least one headgear member is a head band, optionally a single head band.

In some embodiments, the headband comprises a central portion and an end portion(s), the end portion(s) located to a side or a respective side of the user's head, and wherein the central portion is angled with respect to the end portion(s), optionally in-use to fit about a rear potion of the user's head.

In some embodiments, the sliding member comprises at least one passageway, the at least one passageway configured to allow for the passage of the head gear member.

In some embodiments, the headgear member is configured to move or slide within the at least one passageway

In some embodiments, the headgear member comprises at least one stop to limit movement of the headgear member relative to the sliding member and/or the at least one passageway, optionally, the at least one stop is located at an end of the headgear member.

In some embodiments, the at least one stop comprises one or more of:
i. a protrusion
ii. a recess
iii. a barbed end
iv. a localised increase or decrease in thickness, or cross-sectional area of the headgear member, optionally, relative to the size of the at least one passageway.

In some embodiments, the headgear member comprises one or more of a first stop and/or a second stop, wherein the first stop to provide for a first limit of movement of the head gear member relative to the sliding member and/or the at least one passageway, and wherein the second stop to provide for a second limit of movement of the head gear member relative to the sliding member and/or the at least one passageway.

In some embodiments, the at least one headgear member is/are releasably attachable, or connectable and detachable or disconnectable, with or from an adjacent headgear member and/or the sliding member.

In some embodiments, an end of the at least one headgear member extends past or beyond the sliding member, optionally the end of the at least one headgear member configured to rest on a part of the patients head or face in use.

In some embodiments, the sliding member, or the at least one headgear member, comprises a connecting portion or connection arrangement.

In some embodiments, the connecting portion is located to one side of the sliding member, optionally, the connecting portion is located distally from a centre of the sliding member, or the passageway.

In some embodiments, the connecting portion is located on a side of the sliding member opposite the patient's face.

In some embodiments, the connecting portion is located in a lower portion of the sliding member.

In some embodiments, the connecting portion or connection arrangement is configured to provide for connection with one or more of: an interface (or a part of an interface), an arm adapted to connect to or form part of a patient interface.

In some embodiments, the connecting portion or connection arrangement is configured to provide for a pivotal connection.

In some embodiments, the headgear is provided with an interface, and wherein the headgear and interface are configured between a storage configuration and an in use configuration, optionally, wherein in the storage configuration the interface is and headgear are folded together (optionally toward a common plane), and optionally, wherein in the in use configuration the interface is disposed at an angle from the headgear.

In some embodiments, the connecting portion or connection arrangement comprises a recess, or aperture configured to receive a projection or boss, optionally the projection or boss is located on an interface or part of an interface, optionally the recess or aperture is located on the sliding member.

In some embodiments, the connecting portion or connection arrangement comprises a projection or boss configured to be received by a recess or aperture, optionally the projection or boss is located on the sliding member, optionally the recess or aperture is located on an interface or part of an interface.

In some embodiments, the sliding member comprises at least one engagement portion, optionally each of a or the pair of sliding members each comprise at least one engagement portion, and optionally wherein each engagement portion configured to engage a side of the user's head in use.

In some embodiments, the, each engagement portion configured to engage a side of the user's head in use.

In some embodiments, the engagement portions comprise a textured surface, optionally the textured surface comprises one or more of: a knurled portion, a waved surface, a ribbed surface, a roughened surface, or may comprise micro or nano projections, optionally said micro projections is/are about 1x10-6 m, optionally said nano projections is/are about 1x10-9 m. m, optionally said nano projections is/are about 1x10-9 m.

In some embodiments, the engagement portion comprises an adhesive for engagement with the head of the user.

In some embodiments, the adhesive is a biocompatible adhesive.

In some embodiments, the headgear comprises a removable cover over the adhesive to be removed before using.

In some embodiments, the headgear comprises a pair of flexible joints, wherein each arm is attached to the headband by a said flexible joint allowing the arm to move freely relative to the headband with at least two degrees of freedom, and wherein each flexible joint is a said engagement portion.

In accordance with an aspect of the disclosure, there is provided a headgear for a patient interface comprising:
a resilient headband shaped to fit over the top of a user's head with each end of the headband located and biased to a respective side of the user's head, and
a pair of engagement portions, each engagement portion configured to engage a side of the user's head,
wherein in use the headgear has a first lateral distance between the engagement portions, a second lateral distance between left and right side portions of the headband that are intermediate the engagement portions and a sagittal plane position of the headband, and a third lateral distance between ends or end portions of the headgear attached to or attachable to the patient interface,
wherein each lateral distance is across and perpendicular to the sagittal plane, and
wherein the first lateral distance is less than the second and third lateral distances.

In some embodiments, the second lateral distance is greater than the third lateral distance.

In some embodiments, the headgear comprises:
a pair of arms attached to ends of the resilient headband, each arm adapted to connect to or is part of a patient interface, and wherein each arm is attached to the headband by a flexible joint, the flexible joint allowing the arm to move freely relative to the headband with at least two degrees of freedom, and wherein the flexible joints provide or comprise the engagement portions.

In some embodiments, the arms are resilient arms.

In some embodiments, the headband is shaped to fit over the top of a user's head.

In some embodiments, the headband is shaped so that the engagement portions are located in use:
i) in proximity to the temporal bone of the user so that the engagement portions occupy an inward contour in front of the user's ear and above the zygomatic bone or arch when viewed in side profile, or
ii) forward of the ear in proximity to the zygomatic bone when viewed in side profile.

In some embodiments, the headgear comprises at least one positioning tab connected to at least one said headgear member.

In some embodiments, the headgear comprises at least one positioning member connected to at least one said headgear member.

In some embodiments, the patient interface and headgear form a continuous loop comprising an upper portion and a lower portion joined by the pair of flexible joints.

In accordance with a fifteenth an aspect of the disclosure, there is provided a headgear for a patient interface comprising:
a plurality of headgear members connected together to form a continuous loop together with the patient interface in use,
the continuous loop comprising an upper portion and a lower portion, ends of the upper portion and ends of the lower portion connected together by a pair of flexible joints.

In some embodiments, the upper portion comprises a resilient headband shaped to fit a user's head with each end of the headband located to a respective side of the user's head, and
the lower portion comprises a pair of arms, each arm attached to the resilient headband, each arm adapted to connect to or is part of a patient interface,
wherein each arm is attached to the headband by one of the pair of flexible joints.

In some embodiments, the resilient headband is shaped to fit a user's head with each end of the headband located to and biased against a respective side of the user's head.

In some embodiments, each arm is a resilient arm.

In some embodiments, the flexible joint provides for an end portion of the lower portion to move freely relative to a respective end portion of the upper portion with at least two degrees of freedom.

In some embodiments, the flexible joint is or comprises a unitary member.

In some embodiments, the flexible joint is a member formed from a soft flexible material.

In some embodiments, at least one end portion of the lower portion is moveably attached to an end of the upper portion for relative movement therebetween along a longitudinal axis of the lower portion.

In some embodiments, the end portion of the lower portion is telescopically attached to an end of the upper portion.

In some embodiments, the end portion of the lower portion is telescopically received in an end portion of the flexible joint.

In some embodiments, the headgear comprises at least one positioning tab connected to at least one of the upper portion and the lower portion.

In some embodiments, the tab is integrally formed with the flexible joint.

In some embodiments, the headgear comprises a pair of engagement portions, each engagement portion configured to engage a side of the user's head in use.

In some embodiments, the pair of flexible joints provides or comprises the pair of engagement portions.

In some embodiments, in use, the headgear has a first lateral distance between the engagement portions, a second lateral distance between left and right side portions of the headband that are intermediate the engagement portions and a sagittal plane position of the headband, and a third lateral distance between ends or end portions of the arms attached to or attachable to the patient interface,
wherein each lateral distance is across and perpendicular to the sagittal plane, and
wherein the first lateral distance is less than the second and third lateral distances.

In some embodiments, the second lateral distance is greater than the third lateral distance.

In accordance with an aspect of the disclosure, there is provided, a method for donning a headgear and patient interface comprising:
i) applying a lateral force to each side or end of a resilient headband of the headgear against a bias provided by the resilient headband to deform ends of the headband outwards,
ii) positioning the headband over the top of a user's head and the interface to the nose, mouth or nose and mouth of the user,
iii) releasing the lateral force from each side or end of the resilient headband so that the bias of the headband forces engagement portions of the headgear against sides of the user's head to hold the headgear to the user's head, and
iv) manipulating a pair of flexible joints between the headband and a pair of arms of the headgear or between the headband and the patient interface to adjust a relative position between the headband and the patient interface for correct placement of the patient interface on user's nose, mouth or nose and mouth.

In some embodiments, in step (i) the method comprises gripping and pulling a pair of positioning tabs to apply a lateral force to each side or end of the resilient headband against a bias provided by the resilient headband to deform ends of the headband outwards, and
in step (iii) the method comprises releasing the lateral force from each positioning tab so that the bias of the headband forces engagement portions of the headgear against sides of the user's head to hold the headgear to the user's head with the patient interface in position on the user's nose, mouth or nose and mouth.

In some embodiments, each flexible joint is or comprises a said engagement portion, and in step (iii) the method comprises:
releasing the lateral force from each side or end of the resilient headband so that the bias of the headband forces the flexible joints against sides of the user's head to hold the headgear to the user's head.

In some embodiments, in step (iii) the method comprises positioning the engagement portions to be located:
a) in proximity to the temporal bone of the user so that the engagement portions occupy an inward contour in front of the user's ear and above the zygomatic bone or arch when viewed in side profile, or
b) forward of the ear in proximity to the zygomatic bone when viewed in side profile.

In some embodiments, the headband or arm is moveably attached to the flexible joint to move relative to the flexible joint along a longitudinal axis of the headband or arm, and the method comprises:
c) adjusting the position of the headband or arm to the flexible joint for correct placement of the patient interface on user's nose, mouth or nose and mouth.

In some embodiments, the method comprising applying the headband to the head of the user and without applying a secondary headband or strap around the back of the user's head.

In some embodiments, the method comprises applying the headband to the user's head with the user's head resting on a support (such as a pillow) and without lifting the user's head from the support.

In some embodiments, the method comprises applying the headband to the user's head without moving the user's head.

In some embodiments the headgear may be the headgear of any of the first, second, third, fifth, sixth, seventh, eighth, ninth, tenth, eleventh, twelfth, thirteenth, fourteenth, fifteenth or sixteenth aspects.

In accordance with another aspect of at least one of the embodiments disclosed herein, a method of donning a headgear and patient interface, the headgear is the headgear of any of the above aspects.

It will be appreciated that any of the above mentioned features could be combined with any of the other aspects of the invention.

In particular, any of the aspects the first, second, third, fourth, fifth, sixth, seventh, eighth, ninth, tenth, eleventh, twelfth, thirteenth, fourteenth, fifteenth or sixteenth relating to a headgear or patient interface may be interchangeably made operative with the headgear or patient interface of any one of the first, second, third, fourth, fifth, sixth, seventh, eighth, ninth, tenth, eleventh, twelfth, thirteenth, fourteenth, fifteenth or sixteenth aspects.

Further, the connection arrangement or portion as defined in any one of aspects first, second, third, seventh may be provided upon a headgear as defined in any one of aspects first, second, third, fourth, fifth, sixth, seventh, eighth, ninth, tenth, eleventh, twelfth, thirteenth, fourteenth, fifteenth or sixteenth aspects for facilitating connection with a patient interface.

The term 'resilient' in relation to a member or part or material, unless the context suggests otherwise, is intended to mean the member or part or material is able to recoil or spring back into shape after bending or flexing, without permanent deformation or breaking, under normal use conditions. A resilient member or part may be sufficiently stiff to resist bending or flexing under normal use conditions, or may be sufficiently flexible to bend or flex and recoil or spring back into shape under normal use conditions.

The term "comprising" as used in this specification and claims means "consisting at least in part of". When interpreting each statement in this specification and claims that includes the term "comprising", features other than that or those prefaced by the term may also be present. Related terms such as "comprise" and "comprises" are to be interpreted in the same manner.

As referred to in the specification herein, use or reference to the term "living hinge" includes, but is not limited to, straight or flat living hinges and/or butterfly-type living hinges.

As referred to in the specification herein, use or reference to the term 'micro' (such as in relation to a projection or projections) means a dimension that is/are about 1x10-6 m in at least one dimension.

As referred to in the specification herein, use or reference to the term 'nano' (such as in relation to a projection or projections) means a dimension that is/are about 1x10-9 m in at least one dimension.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

As used herein the term "and/or" means "and" or "or", or both.

As used herein "(s)" following a noun means the plural and/or singular forms of the noun.

This invention may also be said broadly to consist in the parts, elements and features referred to or indicated in the specification of the application, individually or collectively, and any or all combinations of any two or more said parts, elements or features, and where specific integers are mentioned herein which have known equivalents in the art to which this invention relates, such known equivalents are deemed to be incorporated herein as if individually set forth.

The invention consists in the foregoing and also envisages constructions of which the following gives examples only.

### BRIEF DESCRIPTION OF THE DRAWINGS

Embodiments are described by way of example only and with reference to the drawings.
**Figure 1** shows a patient interface and headgear.
**Figures 2A to 2D** show a headgear similar to the headgear shown in Figure 1. Figure 2A is a front, top, side perspective view. Figure 2B is a top view. Figure 2C is front view. Figure 2D is a side view.
**Figures 3A to 3D** show the headgear from Figures 2A to 2D attached to a patient interface. Figure 3A is a front, top, side perspective view. Figure 3B is a top view. Figure 3C is a front view illustrating resiliency of a headband of the headgear. Figure 3D is a side view illustrating bending or flexing of flexible joints between headgear members.
**Figures 4A and 4B** show side views of a patient interface with a headgear according to another embodiment.
**Figures 5A** **and** **5B** show a patient interface with a headgear according to another embodiment.
**Figure 5C** shows a headband and second headband.
**Figure 6** shows a patient interface with a headgear according to another embodiment.
**Figures 7A to 7C** show a headgear and patient interface similar to the headgear and interface shown in Figures 3A to 3D, being worn by a user. Figure 7A is a view from a left side and front of the user, Figure 7B is a view on the left side of the user, and Figure 7C is a front view.
**Figure 8** shows a headgear similar to the headgear shown in Figure 1 connected to a full face mask.
**Figure 9** shows a headgear similar to the headgear shown in Figure 1 connected to a patient interface with nasal pillows.
**Figure 10** shows a headgear similar to the headgear shown in Figure 1 connected to a nasal face mask.
**Figures 11 to 11A** show a headgear according to another embodiment.
**Figures 11B to 11C** show a headgear according to another embodiment.
**Figures 12A and 12B** show a headgear according to another embodiment.
**Figures 13A to 13c** show a headgear according to various embodiments.
**Figures 14A-14D** show various connecting portion or connection arrangements.

### DETAILED DESCRIPTION OF EMBODIMENTS

Various embodiments are described below with reference to the Figures.

Throughout the Figures and specification, the same reference numerals may be used to designate the same or similar components, and redundant descriptions thereof may be omitted.

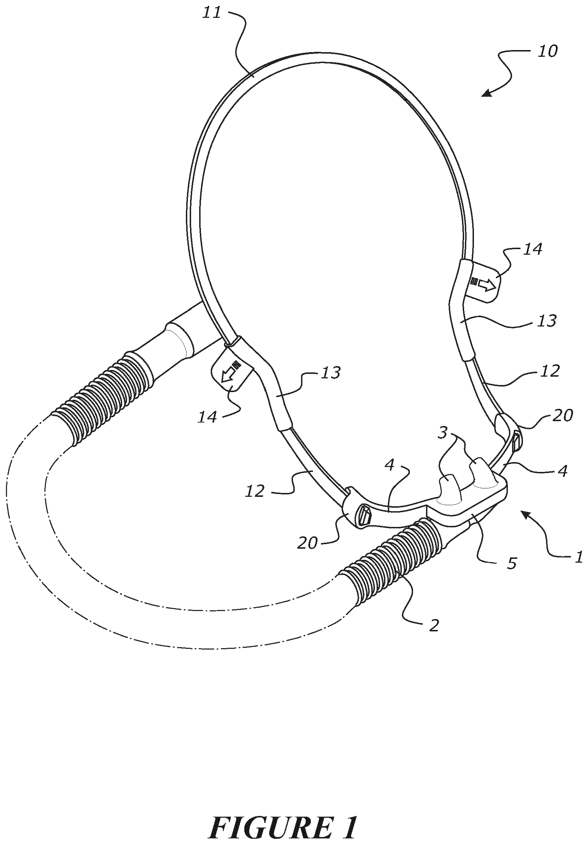

Figure 1 shows a patient interface 1 and headgear 10 according to some embodiments described herein.

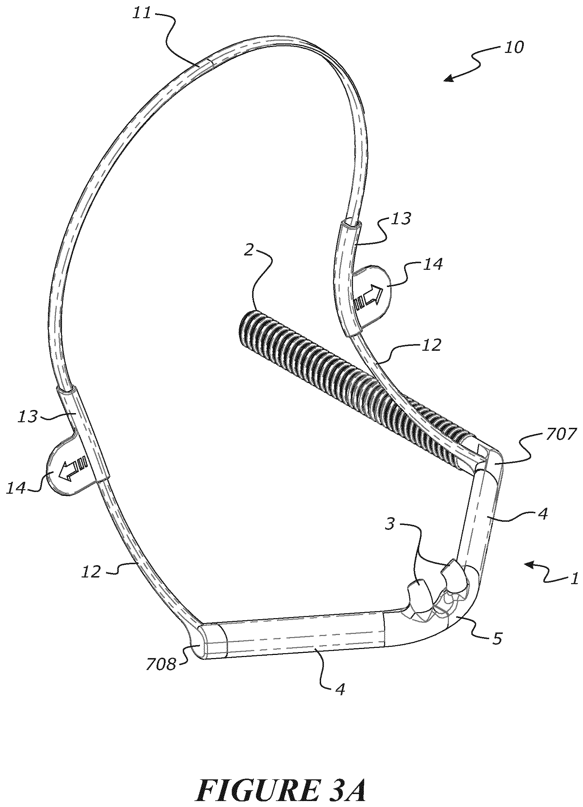

Figures 3A to 3D illustrate a similar headgear attached to an alternative patient interface. The patient interface shown in Figures 3A to 3D is the same or similar to the interface described in US provisional application 62/399893

The patient interface and/or headgear as described in this specification may be used in the respiratory system as disclosed in PCT Application no. WO 2016/157105.

A tube or conduit 2 provides a flow of respiratory gases to the patient via the interface 1.

In some embodiments the patient interface comprises the tube 2. In the illustrated embodiment the patient interface is a nasal cannula comprising a pair of nasal prongs 3. The nasal prongs interface with the nares of a patient or user to provide a flow of respiratory gases to the user.

In the illustrated embodiment the prongs provide a flow of gases to the user without forming a seal with the nares or nasal passages.

In some embodiments the interface 1 may comprise a manifold section 5 comprising an inlet receiving gases from the tube 2 and an outlet or outlets, e.g. the prongs 3. The manifold 5 directs gases received by the manifold from the tube 2 to the prongs 3. The headgear may be attached to either side of the patient interface. In some embodiments the patient interface comprises a side member or arm 4 on each side of the interface. For example, the illustrated example has a side arm 4 extending from each side of the manifold section 5.

In some embodiments, the headgear is attached to each side arm 4, e.g. to an end or end portion of each side arm. A conduit portion may be integrally formed in or with a side member or arm 4 of the cannula.

In some embodiments, a side member or arm 4 is a conduit for transporting a flow of gases from a patient conduit to the manifold section 5. In some embodiments the side arm conduit 4 may comprise a collapsible portion. Substantially a full length of the side arm conduit 4 may be configured to collapse, or a portion of the length of the side arm conduit may be configured to collapse.

In some configurations, patient interface may be adapted to deliver a high flow therapy.

'High flow therapy' as used in this disclosure may refer to delivery of gases to a patient at a flow rate of greater than or equal to about 10 litres per minute (10 LPM). In some configurations, 'high flow therapy' may refer to the delivery of gases to a patient at a flow rate of between about 10 LPM and about 100 LPM, or between about 15 LPM and about 95 LPM, or between about 20 LPM and about 90 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM.

'High flow therapy' may also for example, according to various embodiments and configurations described herein, be a flowrate of gases supplied or provided to an interface or via a system, such as through a flow path, but is not limited to, flows of at least about 5, 10, 20, 30, 40, 50, 60, 70, 80, 90, 100, 110, 120, 130, 140, 150 L/min (LPM), or more, and useful ranges may be selected between any of these values (for example, between about 40LPM to about 80LPM, or between about 50LPM to about 80LPM, or between about 60LPM to about 80LPM, or between about 70LPM to about 80LPM, or between about 5LPM and about 150LPM, or between 10LPM and about 150LPM, or between about 15LPM and about 150LPM, or between about 20LPM and about 150LPM, or between about 20LPM and about 120LPM, or between about 30LPM and about 120LPM, or between about 20LPM and about 100LPM,or between about 20LPM and about 90LPM, or between about 25LPM and about 85LPM, or between about 30LPM and about 80LPM or between about 30LPM and about 90LPM, or between about 35LPM and about 75LPM, or between about 40LPM and about 70LPM, or between about 45LPM and about 65LPM, or between about 50LPM and about 60LPM).

Gases delivered may comprise a percentage of oxygen. In some configurations, the percentage of oxygen in the gases delivered may be between about 20% and about 100%, or between about 30% and about 100%, or between about 40% and about 100%, or between about 50% and about 100%, or between about 60% and about 100%, or between about 70% and about 100%, or between about 80% and about 100%, or between about 90% and about 100%, or about 100%, or 100%.

In some embodiments, the headgear 10 comprises at least one headgear member, for example a headband 11, and a pair of flexible joints 13 adapted to connect ends or end portions of a headgear member or a pair of headgear members to a patient interface. Each flexible joint 13 allows relative movement between the headgear member and the patient interface with at least two degrees of freedom.

In some embodiments, each flexible joint 13 allows free relative movement between the headgear member and the patient interface with at least two degrees of freedom.

Unless the context suggests otherwise, 'free movement' or to 'move freely' means the flexible joint does not employ a mechanism such as friction or mechanical indexing to impede movement or set or lock the flexible joint in a particular position. The flexible joint may allow free movement with at least 2 degrees of freedom between limits of movement. Limits of movement may be provided by the headgear members, for example by material choice and/or geometry.

In some embodiments, the headgear 10 comprises a plurality of headgear members 11, 12 connected together. For example, the headgear may comprise a headband 11 and a pair of arms 12 attached to the headband. Each arm 12 may be adapted to connect to a patient interface 1. In the illustrated embodiment, each arm 12 facilitates connection of the headgear 10 to the patient interface. The arms 12 act as a connecting structure between the interface 1 and the headband 11.

The headband is a resilient headband. In some embodiments the arms 12 are resilient arms.

Adjacent headgear members 11, 12 are attached or connected together by a flexible joint 13.

In some embodiments the headgear members are integral with the flexible joint 13.

In some embodiments the at least one headgear member is/are releasably attachable, or connectable and detachable or disconnectable, with or from an adjacent headgear member and/or the flexible joint. In some embodiments an end of the at least one headgear member extends past or beyond the flexible joint 13, which can increase contact with a user's head when in use for better pressure distribution

As described above, the flexible joint 13 allows for one member (e.g. arm 12) to move freely relative to the adjacent connected member (e.g. the headband 13).

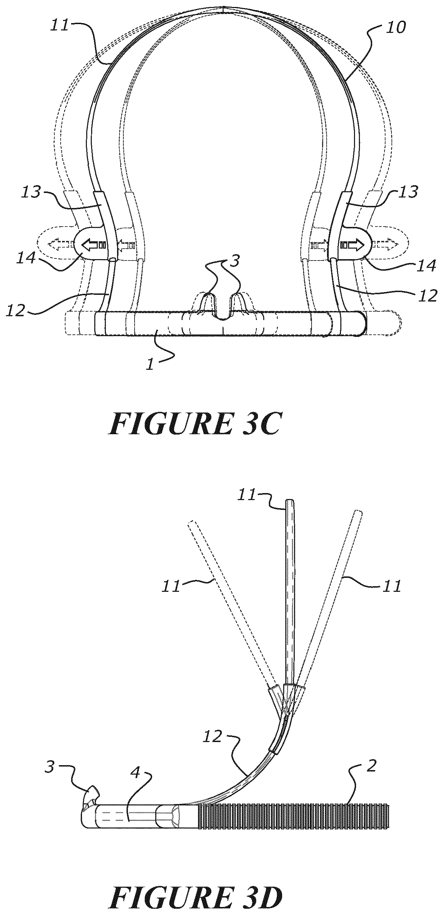

In some embodiments, the flexible joint 13 allows relative movement between adjacent headgear members with at least two degrees of freedom. For example, in Figure 3D, the headband 11 is shown to move in a direction relative to the arms 12, that is a front-and-back direction, and the flexible joint may also allow each arm 12 to move relative to the headband in a left side to right side direction (i.e. perpendicular to the page), presenting two degrees of freedom of relative movement between the members 11, 12.

In some embodiments the flexible joint 13 may be located at or near the sides of a user's heard, and/or at or near the top of a user's head.

In some embodiments the flexible joint is located at or near a central portion of a headband.

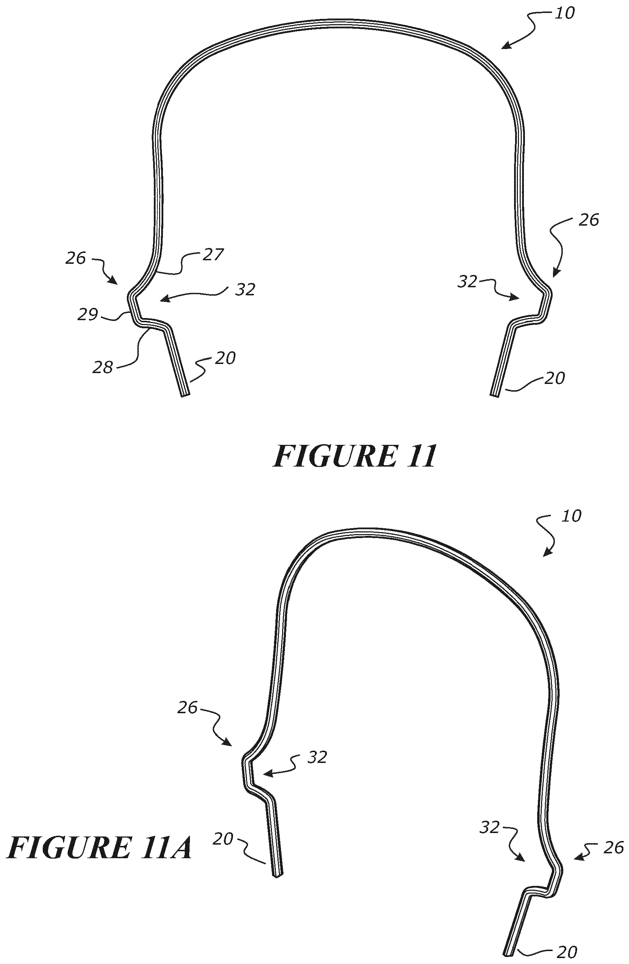

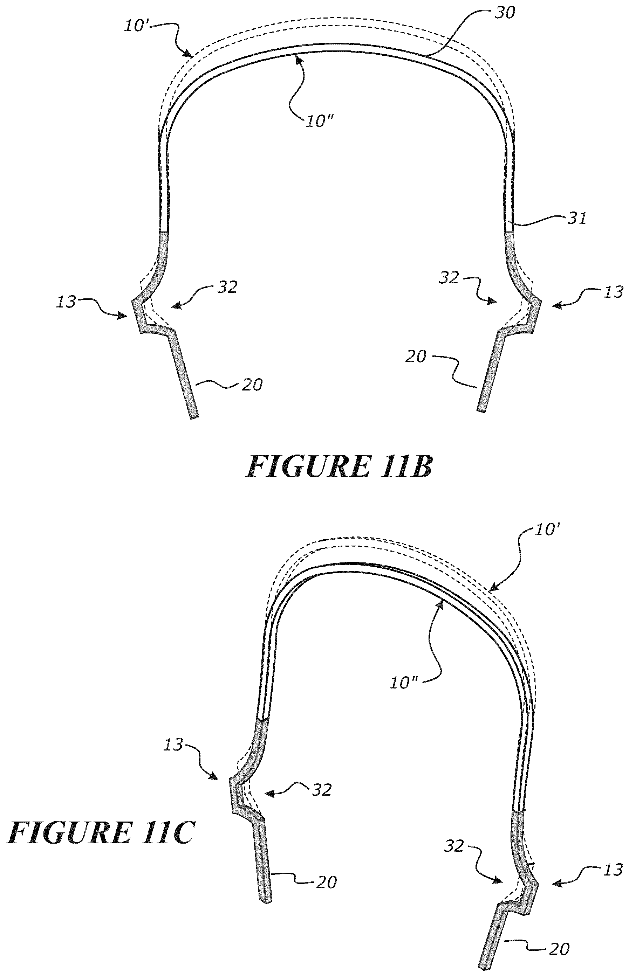

In some embodiments, the flexible joint 13 allows relative movement between adjacent headgear members with at least one degrees of freedom. In some embodiments, for example as shown in Figures 11B-11C, the flexible joint 13 may provide for elongation optionally in a lengthwise direction. The elongation of allows for movement between adjacent headgear members in a direction away from each other. Elongation of the flexible joint 13 can help to size the headgear for a particular patient.

In some embodiments, for example as shown in Figures 11B-11C, the flexible joint 13 may be provided by or comprise a substantially u-shaped portion. The flexible joint may have a first portion extending outward from a headgear member in use (e.g. outwardly from the user's head), and a second portion extending toward a headgear member in use (e.g. toward from the user's head).

In some embodiments the flexible portion 13 may have an intermediate portion located intermediate the first portion and the second portion, the intermediate portion extending substantially parallel to a headgear member in use (e.g. parallel to the user's head).

In some embodiments, for example as shown in Figures 11B-11C the flexible portion may be integrally formed as part of the headgear member, and/or headband.

In some embodiments, the flexible portion may be formed of the same material as the adjacent headgear members but be geometrically shaped to have a greater stiffness in one particular plane relative to one or more other planes. In some embodiments the flexible joint is stiffer in a coronal plane of the user, or a plane of the headband, as compared to the sagittal and medial planes of a user (or the other orthogonal planes).

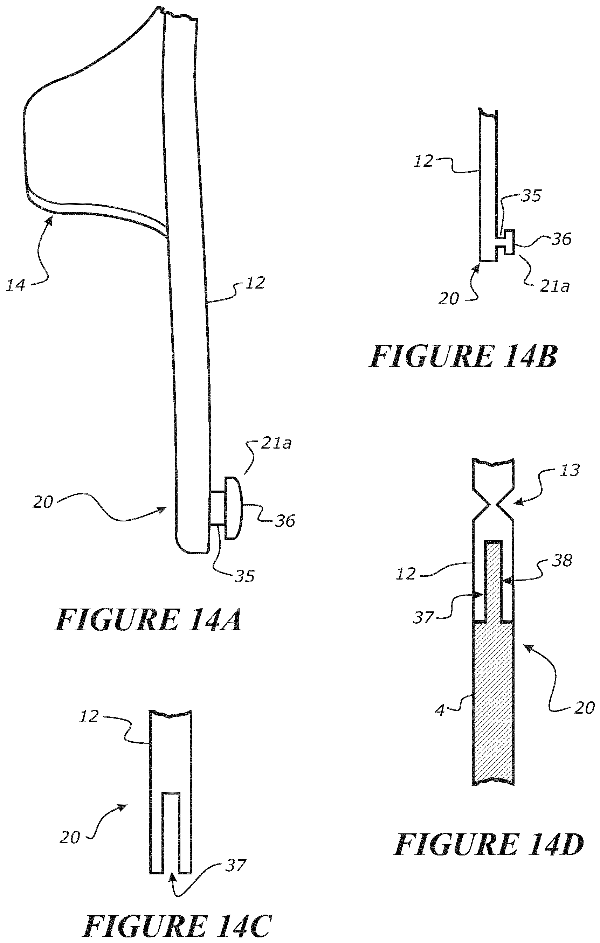

In some embodiments for example as shown in Figure 14D the flexible joint 13 may comprise a narrowed portion optionally to form a hinge. The narrowed portion may comprise one or more rounds, bevels or substantially V-shaped portions, to narrow the at least one headgear member from a first cross-sectional area to a second cross-sectional area which is smaller than the first cross-sectional area.

In some embodiments the flexible joints may comprise a relatively flexible material relative to the headgear members (i.e. rubber or silicon) and/or a concertina or bellows section and/or a spring section so as to allow for relative movement by elongation of the flexible joint, and preferably at least some relative movement of connected headgear members away from each other.

In some embodiments the flexible joint may allow for relative movement of around 5mm to around 20mm.

In some embodiments the flexible joint may comprise at least one, or a plurality of sub sections.

In some embodiments each sub section is a flexible joint as described elsewhere in the specification. In some embodiments one or more sub section(s) may provide for flexibility or free relative movement in one or more directions or degrees of freedom. For example, the flexible joint may comprise one or more living hinges, where each living hinge provides for flexibility or free relative movement in a degree of freedom.

In some embodiments one or more sub section(s) may provide for elongation (optionally lengthwise or in a direction along the at least one headgear member) to allow free relative movement between the headgear member or members and the patient interface or between the headgear member and adjacent headgear members.

In some embodiments the flexible joint may comprise so one or more: living hinge, or concertina, or bellows section, or spring section.

In some embodiments, the flexible joint 13 is adapted to bend or fold laterally to a longitudinal axis of an end portion of a said headgear member, e.g. the headband 11. The joint may bend or fold/flex in any direction perpendicular to a longitudinal axis of an end portion of the headgear member (2-degrees of freedom). The flexible joints 13 may allow the headband to be folded over onto the arms 12 to provide a folded up non-use configuration, for example in a package for sale.

In some embodiments, the flexible joint is or may comprise a flexible unitary member, for example a hollow member or a tubular member, e.g. a length of tube. The flexible joint may be a formed from a soft flexible material, such as an elastomeric material. The flexible joint may be a length of elastomeric tube, for example silicone tube or tubular member. The flexible joint preferably elastically deforms (without permanent deformation) under normal use conditions, so that the joint may flex between different positions many times without changing mechanical properties.

In some embodiments, the flexible joint may be over-moulded to the headband 11, or may be assembled to the headband.

In some embodiments the flexible joint may be provided by a mechanical assembly or mechanism, for example a universal joint, or a two way hinged assembly allowing for hinging about two perpendicular axes. A hinge may be a living hinge.

In some embodiments, the flexible joint allows for rotation of a said headgear member, e.g. arm 12, relative to an adjacent headgear member, e.g. band 11, about a longitudinal axis of an end portion of the headgear member 12 attached to the adjacent headgear member 11. This rotation may be provided for by torsional elastic deformation of the flexible joint, e.g. twisting of the flexible joint. This torsional deflection presents a further freedom of movement, such that in some embodiments the flexible joint presents three degrees of freedom of movement.

In some embodiments, the flexible joint simultaneously accommodates lateral bending/folding in two degrees of freedom and also torsional rotation, to allow the joint 13 to flex in multiple dimensions. The flexible joints 13 take up or allow for torsional forces and bending resulting from movement of the arms 12 and/or interface 1 relative to the headband 11.

In some embodiments, the flexible joint provides bias against movement from an un-deflected or 'normal' position. For example, in some embodiments, the flexible joint is or comprises a flexible member such as an elastomeric member. The elastomeric member presents an un-deflected shape or relative position between adjacent members connected together by the flexible member. Movement between the connected members is achieved by flexing the flexible member away from the un-deflected position and resiliency of the flexible member acts to bias the flexible joint to the un-deflected position. A mechanical flexible joint may comprise a resilient member, e.g. a spring, to bias the joint to an un-deflected position.

In some embodiments the flexible joint may comprise a bellows or concertina section.

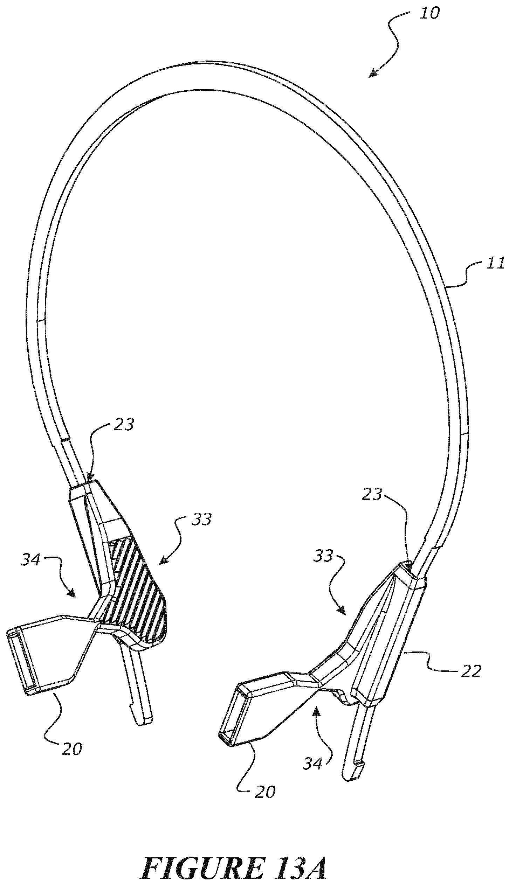

In some embodiments the flexible joints 13 may be absent and the headband 11 may be a continuous rigid headband. Such arrangements are shown in Figures 11-11A and 13A-13C which show continuous headbands 11. However, as is discussed in more detail below these arrangements may comprise a single piece headband, a rigid or continuous headband with flexible joints 13 (for example as shown in Figures 11B and 11C).

In some embodiments the headgear comprises at least one connecting portion or connection arrangement 20. The connecting portion or connection arrangement 20 may be located on, or form part of the flexible joint(s) 13, or the at least one headgear member (for example band 11 or arm 12). The connecting portion or connection arrangement 20 may be configured to provide for connection with one or more of: an interface (for example interface 1, or any of the interfaces as shown in Figures 1, 3A-13C), or a part of an interface, an arm (for example side arm 4) adapted to connect to or form part of an interface.

Optionally, when the connecting arrangement 20 is located on the at least one headgear members, the connecting arrangement 20 may be located away from an end of the at least one headgear member so that the headgear member extends past the connecting arrangement 20 to optionally rests on the user's face.

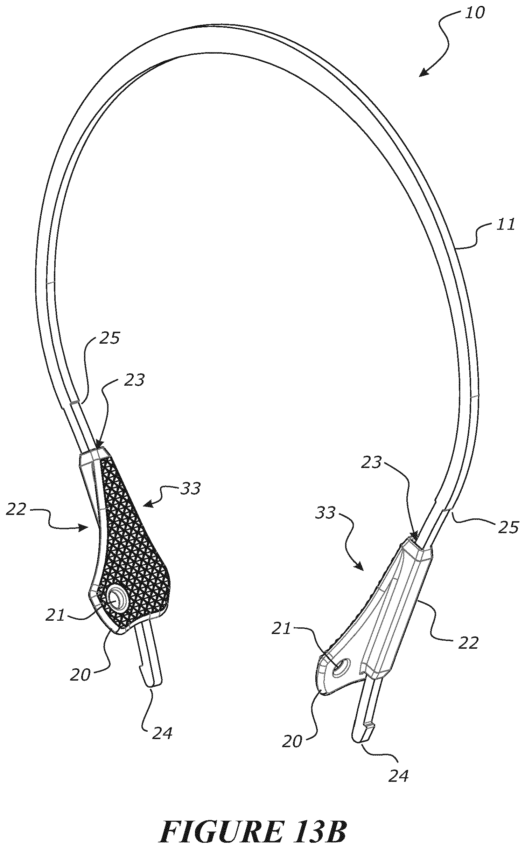

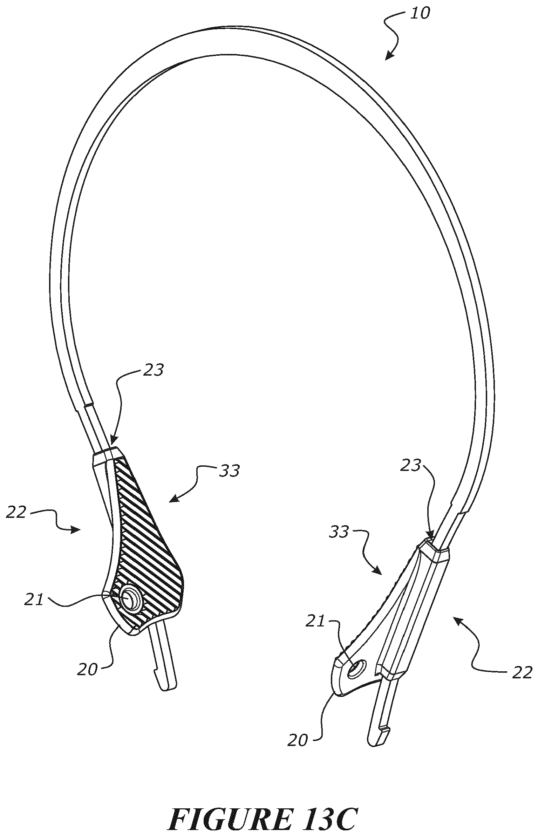

As shown in Figures 13B and 13C, and Figure 14A-14B the connecting portion or connection arrangement 20 may be configured to provide for a pivotal connection, or a rigid or semi-rigid connection. The connecting portion or connection arrangement 20 may comprise a recess, or aperture 21 configured to receive a projection or boss 21a, optionally the projection or boss 21a may be located on an interface 1 or part of an interface, optionally the recess or aperture is located on, or as part of, the flexible joint 13, or on a sliding member 22 of the headgear 10.

In some embodiments the projection or boss may comprise a first portion 35 which has a smaller diameter or cross-sectional area than a second portion 36. The second potion 36 may be configured to retain the projection or boss 21 within recess or aperture 21.

Alternatively, the connecting portion or connection arrangement 20 comprises a projection or boss configured to be received by a recess or aperture. Optionally, the projection or boss 21a is located on the flexible joint 13 or on a sliding member 22 of the headgear 10, optionally the recess or aperture is located on an interface or part of an interface.

In some embodiments, the connecting portion or connection arrangement 20 may comprise of a slot 37 and a projection 38. The slot 37 or projection 38 may be located on, or form part of the flexible joint(s) 13, or the at least one headgear member (for example band 11 or arm 12) with the remaining part (i.e. the projection 38 or slot 37) may be located on one or more of: an interface (for example interface 1, or any of the interfaces as shown in Figures 1, 3A-13C), or a part of an interface, an arm (for example side arm 4) adapted to connect to or form part of an interface. Such a connection arrangement 20 is shown in Figures 14C and 14D. The slot 37 and projection 28 may be connected by one or more connection features, or any adhesive, or alternatively by over moulding.

In some embodiments the connection portion or connecting arrangement 20 may be one or more of: a Snap-fit, lock/key arrangement, male/female connection, or a plug socket arrangement.

In some embodiments the arm of the interface, the flexible joint, and/or the sliding member of headgear may comprise one or more corresponding connection portions or arrangements. Optionally, multiple corresponding connection portions or arrangements may be provide on the arm of the interface, the flexible joint, and/or the sliding member of headgear to provide for various sizings, for example adjustments along the height, e.g. a distance from the tip of the chin to the level of the top of a user's head, and/or depth, e.g. anterior to posterior of a user's head.

It will be appreciated that in Figures 11C-12B the connecting portions 20 are not shown however an example of a location of the connecting portions 20 is shown. It will be appreciated that these embodiments could be modified to include the connecting portions 20 as described above.

In some embodiments the headgear and an interface, or a part of an interface, or an arm adapted to connect to or form part of an interface may be integrally formed.

In some embodiments, a headgear member 11, 12 may be moveably attached to the flexible joint to move relative to an adjacent headgear member along a longitudinal axis of an end portion of the headgear member 11, 12 attached to the flexible joint.

In some embodiments, the headgear member is telescopically attached to the flexible joint. One or both arms 12 may be movably attached to the respective flexible joint 13 to allow the arm 12 to move along a longitudinal axis of the end portion of the arm attached to the flexible joint. The arm or member may be received within a bore or recess of the flexible joint to slide therein to form a telescoping engagement.

An embodiment of such a telescopic arrangement is shown in Figures 13A-13C, these figures show a sliding member 22 being configured to slide relative to the headgear member (for example headband 11, or end 12a of resilient arm 12.) In some embodiments there may be a pair of sliding members 22. The sliding member 22 is or optionally comprises flexible joint (as described above.) The sliding member 22 may comprise at least one passageway 23. Optionally, sliding member 22 is or comprises a hollow or tubular member, which may optionally form said passageway 23. The at least one passageway 23 may be configured to allow for the passage of the headgear member. The headgear member may be configured to move or slide relative to the sliding member 22.

In some embodiments an end of the at least one headgear member extends past or beyond the sliding member 22. In some embodiments the end of the at least one headgear member may be configured to rest on a part of the patients head or face in use.

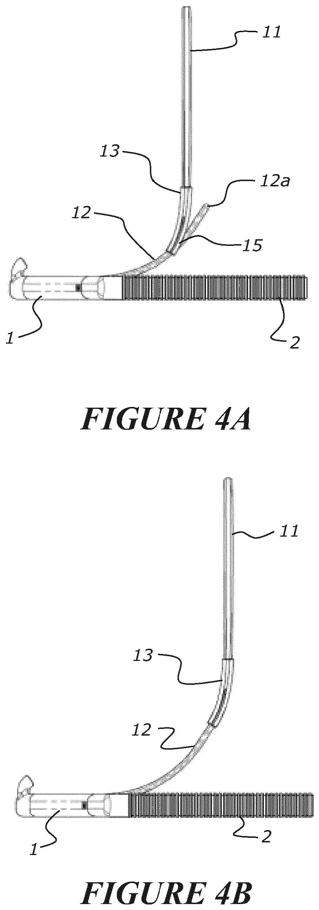

As shown in Figure 4A, in some embodiments, the flexible joint may include an opening or openings, each opening (15 in Figure 4A but obscured from view) allowing an end of the headband 11 or end 12a of resilient arm 12 to extend through a side of the flexible joint. The relative position of the headgear members may be adjusted by pushing the headgear member through an opening to position the flexible joint a desired longitudinal position along the headgear member, or by positioning the arm within the flexible joint, as shown in Figure 4B. Relative movement between the headgear member and the flexible joint allows for size adjustment of the headgear to accommodate different sized users. Size adjustment can be in an anterior direction and/or can also be in the superior or vertical direction.

As shown in Figures 13A-13C, the headgear member may comprise at least one stop 24, 25 to limit movement of the headgear member relative to the sliding member 22 and/or the at least one passageway 23. The at least one stop, may be located at or near an end of the headgear member (for example stop 24), and/or along a length of the headgear member (for example stop 25).

The at least one stop may comprise one or more of:
i. a protrusion
ii. a recess
iii. a barbed end (for example as shown by stop 24).
iv. a localised increase or decrease in thickness, or cross-sectional area of the headgear member, optionally, relative to the size of the at least one passageway (for example as shown by stop 25).

In some embodiments headgear member (for example headband 11) comprises at one or more of: a first stop 24, and a second stop 25. The first stop 24 may provide for a first limit of movement of the head gear member relative to the sliding member 22 (which may optionally be a flexible joint) and/or the at least one passageway 23. The second stop 25 may provide for a second limit of movement of the head gear member relative to the sliding member 22 (which may optionally be a flexible joint).

In some embodiments the flexible joint may allow for relative movement (optionally by elongation of the flexible joint) of around or about 5mm to around or about 20mm. in some embodiments, each flexible joint provides for stepped telescopic movement in increments of about 5mm, for example via engagement and disengagement of notches in, along and/or on the passageway 23.

In some embodiments the sliding member 22 comprises a connecting portion 20 as described elsewhere in the specification. In some embodiments, for example as shown in Figures 13A-13C, the connecting portion 20 may be located to one side of the sliding member 22, optionally, the connecting portion 20 is located distally from a centre of the sliding member 22, or the passageway 23. The connecting portion 20 may be located on a side of the sliding member 22 opposite the patient's face. In some embodiments the connecting portion is located in a lower portion of the sliding member.

The headgear may be provided with an interface, and the headgear and interface may be configurable between a storage configuration, and an in use configuration.

In some embodiments the interface may pivot about the headgear via connection portion 20 so the headgear is folded towards the headband 11. This provides for a storage configuration with a smaller profile. In some embodiments the interface may pivot and/or hinge about the headgear via flexible joint 13 so the headgear is folded towards the headband 11. The headband 11 and interface may be folded towards a common plane, so that in a storage configuration the interface and headgear may be folded in a common plane. The in use position may be that shown in any of Figures 1-10 where the interface is disposed at an angle from the headgear.

In some embodiments the sliding member 22 may comprise an engagement portion 33 as described below. The sliding member 22 may have at least one engagement portion. In some embodiments each of a, or the, pair of sliding members 22 each have an engagement portion 33.

In some embodiments the sliding member may be made of a stiffer material than the at least one headgear member, and/or the headband. In some embodiments the sliding member may be made of a more flexible material than the at least one headgear member, and/or the headband.

In some embodiments the sliding member may be configured to deform the profile of the headgear member which passes through the passageway to provide for a predetermined profile of a section of the headgear member in the area which passes through the passageway. Optionally this section is a straight section, or a curved section.

The longitudinal movement may be indexed or otherwise settable to set the longitudinal position of the headgear member to the flexible joint. In such an embodiment the longitudinal position may be set so that the member substantially does not move freely relative to the flexible joint.

In some embodiments, the headgear member may comprise protrusions or depressions (e.g. dimples or grooves) to be received by corresponding depressions or protrusions of the flexible joint, to provide an indexing arrangement to set the longitudinal position of the member to the flexible joint.

In some embodiments the headband and/or arms may be marked with size graduations, which may align with a corresponding indication or other feature on the flexible joint.

In some embodiments, the each one of the pair of flexible joints engages a respective side of the user's head in use.

In some embodiments, the headband is shaped to fit over the top of a user's head with each end of the headband located to a respective side of the user's head.

In some embodiments, the headband 11 is a resilient headband shaped to fit a user's head with each end of the headband located to a respective side of the user's head, and each flexible joint is attached to a respective end or end portion of the headband.

In some embodiments, the pair of flexible joints 13 provide or comprise a pair of engagement portions 33, each engagement portion 33 configured to engage a side of the user's head in use.

As described above, in some embodiments the flexible joints may be adapted to attach directly to a patient interface, or may connect adjacent headgear members together, like members 11 and 12 shown in Figure 1. In Figure 1, the arms 12 are removably coupled to the interface 1. The arms 12 directly couple to the side arms 4 of the interface 1, e.g. to ends of the arms 4. Each arm 12 couples to one lateral end of the interface and the lateral ends are laterally opposed to each other. In some embodiments, one or both arms 12 may be a resilient arm.

In some embodiments, the headband is shaped to fit over the top of a user's head. In some embodiments, the headband is shaped so that the flexible joints connecting the arms to the headband are located in use:
i) in proximity to the temporal bone of the user so that the flexible joints occupy an inward contour in front of the user's ear and above the zygomatic bone or arch when viewed in side profile, or
ii) forward of the ear in proximity to the zygomatic bone when viewed in side profile.

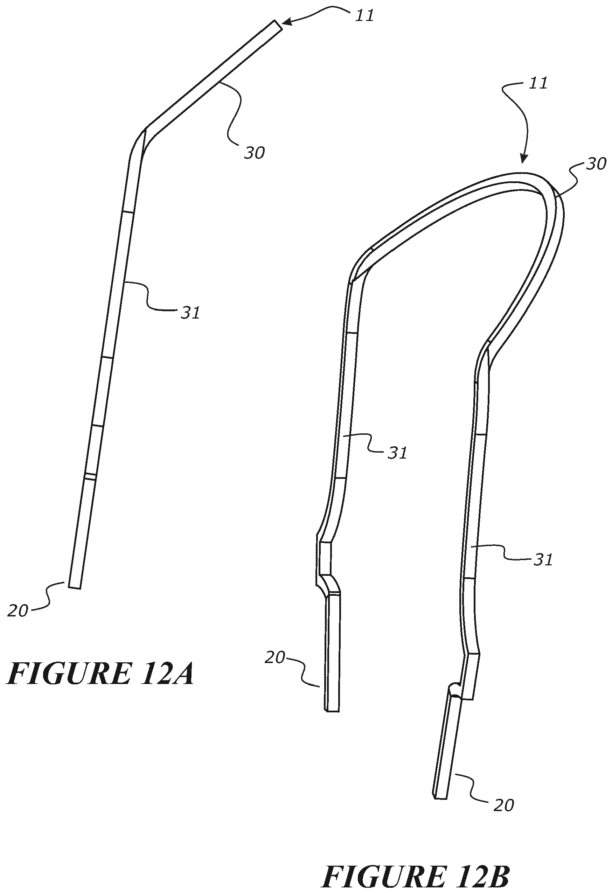

Referring now to Figures 12 and 12A where headband 11 comprises a central portion 30 and an end portion(s) 31. In use the end portion(s) may be located to a side or a respective side of the user's head. The central portion 30 may be angled with respect to the end portion(s), optionally, to fit about a rear portion of the user's head in use. The central portion 30 adapted to fit about a rear portion of a user's head in use, allows the headband to fit a wide range of user head sizes.

In some embodiments the headband is a resilient band, e.g. a headband formed from a resilient material. One or both arms may be resilient arms, and may be formed from the same resilient material as material of the headband.

The resilient headband is shaped to fit a user's head with each end of the headband located to and biased against a respective side of the user's head.

In an un-deflected condition the distance between the ends of the headband is less than a width of a user's head so that deflection of the headband to fit the user's head biases the ends of the headband against sides of the user's head in use.

The headband is of a sufficient length relative to its cross section to be flexible enough to allow a user to spread apart ends of the headband by a user and to provide a comfortable amount of force against the sides of a user's head. The amount of bias provided by the headband provides a force to the user's head that is comfortable yet assists to maintain a correct position on the user's head.

In some embodiments, the geometry, e.g. the length and cross sectional area, of the arms 12 may prevent significant flexing of the arm in normal use.

The arm 12 may be sufficiently short relative to its cross sectional area to resist bending laterally or twisting.

In the illustrated embodiments of at least Figures 1 to 6 the arms 12, although may be made from the same material as the resilient headband 11, are rigid (e.g. do not flex substantially under normal use conditions).

In particular the arms 12 are rigid when the interface is substantially soft and flexible like in the embodiments of Figure 3A - 6. Rigid arms 12 assist in maintaining a robust connection between headband 11 and the interface 1, and arms 12 function to effectively transfer forces from the interface 1 to the flexible joints 13.

In some embodiments a central portion 30 of the headband 11 is relatively stiffer or more stiff or less flexible, than the end portions 31. The central portion 30 may have a larger cross-sectional area than the end portions 31. The central portion 30 may have a cross-sectional area different than the end portions 31.

In some embodiments, the central portion 30 is made of a first material and the end portions 31 are made of a second material, and the first material is relatively stiffer or more stiff or less flexible, than the second material. In some embodiments the relative stiffness of the central portion 30 to the end portions 31 may be provided a particular direction for example in a direction towards a user.

The resilient headband and/or resilient arms may have a rectangular cross section, a trapezoidal cross section, a circular cross section, or any other suitably shaped cross section.

Preferably a cross section with a flat surface is adopted to provide sufficient contact area on the user's head so that sufficient friction is present to retain the headband in a desired position. The flat face will sit against the head and/or face of the user. The cross section of the headband and/or arms may change along their lengths, for example widening, or narrowing in order to provide greater or lesser dispersion of forces about regions of the patient's face and/or head.

In some embodiments, the modulus of elasticity (Young's modulus) of at least one of the headgear members is at least about 0.5GPa, or at least about 1GPa or at least about 1.5GPa, or at least about 1.8GPa, or at least about 2GPa, or at least about 3GPa. For example the headband or the headgear and arms may be formed from polymers which include but are not limited to rubbers (natural and synthetic) and plastics such as acrylonitrile butadiene styrene (ABS), polyethylene terephthalate glycol (PETG) and polypropylene (PP) which has a modulus of elasticity of about 1GPa to 3GPa. In some embodiments the headband or headband and arms may be formed from a metal material, for example a high tensile steel. Thus the Young's modulus of the headgear members may be 200GPa or even higher.

In some embodiments the headband or the headgear and arms may be formed from a resilient material which is not substantially brittle.

In some embodiments, one or more of the headgear members, e.g. the headband 11 and/or the arms 12, may comprises a plastic core and a textile casing, wherein the plastic core and the textile casing are formed as an integral structure by the application of a molten plastic material into the textile or natural fibre casing or a composite of textiles and natural fibres, for example as described in PCT application published as WO2016/043603.

The textile may be a synthetic textile or a textile made from natural fibres. Examples of natural fibres include wool, jute and flax. The plastic core of the headgear member may provide a resiliency so that the member is a resilient member. Additionally or alternatively a resilient member such as a metal band may be provided in or together with the plastic core to provide sufficient resiliency.

In contrast to the headgear members, e.g. members 11 and 12, the flexible joint may be formed from a resilient material with a significantly lower modulus of elasticity. For example, the flexible joint may be formed from a material with a modulus of elasticity of less than about 0.2GPa, or less than about 0.1GPa, or less than about 0.06GPa. An example material is silicone rubber, which may have a Young's modulus of 0.05GPa or less. Thus, in some embodiments the headgear members are formed from a relatively stiff resilient material and the flexible joint is formed from a correspondingly relatively soft flexible material, wherein the modulus of elasticity of the stiff resilient material is many times greater than the modulus of elasticity of the soft flexible material. The soft flexible material is resilient in that it deforms without plastic deformation under normal use conditions. However, it is much more flexible than the material of the headgear members.

In some embodiments, the modulus of elasticity of the stiff resilient material of the headgear members is at least 10 times the modulus of elasticity of the soft flexible material of the flexible joints, or at least 20, or 40, or 100, or 200 times the modulus of elasticity of the soft flexible material.

In some embodiments, the flexible joints 13 isolates or separates movement of the interface from the headband, so that the headband is less likely to be disturbed on the head of the user.

In some embodiments, the headgear is mostly held to the user's head by the headband. The flexible joints 13 may act to isolate the lower portion of the headgear and/or interface to avoid or reduce disturbance of the bias toward the sides of the user's head provided by the headband.

In some embodiments, at least one positioning tab 14 or positioning member 26 is connected to, or forms part of, at least one said headgear member. For example, in some embodiments the headgear has a pair of positioning tabs 14 or positioning members 26, each tab or member located at or near to an end or end portion of the headband 11. The at least one positioning tab or positioning member may allow a user to apply and remove said headgear.

In some embodiments the tabs or members are arranged to be located at a side of the user's head in use.

In some embodiments, the positioning tab or positioning member 26 is provided on one of the flexible joints 13 described earlier. For example, the tab 14 or member 26 may be integrally formed with the flexible joint.

In some embodiments, the least one positioning tab 14 or positioning member 26 is connected to, or integrally formed with a headgear member. The flexible joint with tab 14 or member 26 may be a unitary member.

In some embodiments, the tabs 14 or members 26 are provided with indicia to illustrate a direction to pull the tab to remove the headgear from a user's head, for example the tabs or members may include arrows marked on or formed in the tabs, as illustrated in the figures, the arrow indicating the direction to pull the headgear for removal. In some embodiments the tabs or members may extend from the headgear member so that in use the tabs protrude or extend outwardly from the user's head.

As shown in Figures 11-11C, in some embodiments the at least one positioning tab 14 or positioning member 26 may be provided by a substantially U-shaped portion. Optionally, the positioning tab 14 or positioning member 26 comprise a first portion 27 extending outward from a headgear member in use (e.g. outwardly from the user's head), and a second portion 28 extending toward a headgear member in use (e.g. toward from the user's head).

In some embodiments, the positioning tab 14 or positioning member 26 comprise an intermediate portion 29 located intermediate the first portion 27 and the second portion 28, the intermediate portion 29 may extend substantially parallel to a headgear member in use (e.g. parallel to the user's head). Optionally the geometry of the positioning member can provide for the channel 32 as described below.

Figure 3C illustrates deflection of the headgear 10 and patient interface 1 in a direction lateral to the sagittal plane of a user. A user or medical practitioner may pull on the tabs, e.g. by gripping the tabs between thumb and forefinger, to apply and remove the headgear. Once the headgear is placed on the user's head, the medical practitioner may use the tabs to reposition the headband, for example by adjusting an angle of the headband relative to the arms 12 to better support or position the patient interface and/or improve patient comfort.

In some embodiments such as those shown in Figures 11B and 11C, the headband 11 may comprise one or more flexible joints 13. Figures 11B and 11C illustrate deflection of the headgear 10 in a direction along the coronal plane (i.e. perpendicular to the sagittal plane of a user). In other words, Figures 11B and 11C illustrate deflection of the headgear 10 in a direction from the chin towards the forehead of a patient, or from the forehead towards the chin of the patient. The solid line section 10" shows the original un-deformed shape of the headgear 10. The dashed line section 10' shows the deformed shape of the headgear when the headgear is stretched in a substantially coronal plane (for example when it is placed on or fitted to a user). The interface can be fitted to a user by stretching the flexible joint 13 to allow for the headgear to be deformed to match the shape of the head of a user. Once the stretching force is removed the headgear may return towards its un-deformed shape 10" to engage with a head of a user.

In some embodiments when a force is applied to the headgear to fit the headgear and/or interface to a user's head, the shape of the headgear changes from a generally tall oval to a rounded rectangle shape.

In some embodiments positioning tabs 32 may be provided as part of the, or separately to, the flexible portion 32 these may assist a user to fit the headgear as is described elsewhere in this specification.

In some embodiments the user may pull on or twist the tabs or members to apply a torsional force to the headgear members to twist the headgear member so as to open the headgear (e.g. in a direction along the sagittal plane of a user's head in use) so it can be applied or removed from a user's head. The headgear members and/or the flexible joints may be configured to deform torsionally when a force is applied to the tabs or members. Applying a force (e.g. pulling) on the tabs or members may elastically deform the headgear (for example one or more of a flexible joint, a headband, an arm, or any other headgear member). Once the force is removed the headgear may return towards an initial resting state whereby it engages with the head of a user. In some embodiments there may be no flexible joint (e.g. in Figures 11, 11A, 12 and 12A where the headband 11 could be configured to connect directly to a patient interface), or the flexible joint may be configured to deform torsionally.

In some embodiments the headband may be made from a material such as PETG (Polyethylene Terephthalate with a glycol modification).

In some embodiments the headgear comprises a pair of engagement portions 33, each engagement portion 33 configured to engage a side of the user's head in use.

In some embodiments each engagement portion 33 frictionally engages a side of the user's head. One or more engagement portions 33 may be integral with the headgear, or part thereof, and/or the at least one flexible joint, and/or be removable separate portions. The engagement portions 33 may provide friction pads to engage the user's head, and improve contact with the user's face. In some embodiments the engagement portion 33 comprises a textured surface to contact the head of the user, to increase friction between the headgear and a user's head to assist with maintaining the headgear in position on the user's head.

In some embodiments the textured surface may comprise one or more of: a knurled portion, a waved surface, a ribbed surface, a roughened surface, or may comprise micro or nano projections.

In some embodiments, the engagement portion may 33 comprise an adhesive for engagement with the head of the user. The adhesive may be a biocompatible adhesive. The headgear may comprise a removable cover over the adhesive to be removed before user, e.g. a peelable layer.

In some embodiments, the pair of flexible joints 13 is or comprises the pair of engagement portions 33, each one of the pair of flexible joints configured to engage a side of the user's head in use. For example a textured pad or surface may be provide on an inwardly facing surface of the flexible joint to engage the side of the user's head.

In some embodiments, the headgear members 11, 12 are connected together to form a continuous loop together with the patient interface in use. With reference to Figure 1 as an example and going around the loop from the interface 1, the continuous loop is formed by the manifold section 5, interface side arm 4, headgear arm 12, flexible joint 13, headband 11, flexible joint 13, headgear arm 12 and interface side arm 4. The continuous loop comprises an upper portion and a lower portion with ends of the upper portion and ends of the lower portion connected together by the pair of flexible joints 13.

In some embodiments, the upper portion is provided by the headband 11, and the lower portion by the headgear arms 12 and patient interface 1.

As described earlier, in some embodiments the arms and headband may be moveably attached together for relative movement therebetween along a longitudinal axis of the end portion of the arm. Thus, in some embodiments, at least one end portion of the lower portion may be moveably attached to an end of the upper portion for relative movement therebetween along a longitudinal axis of the end portion of the lower portion. This movement allows for the length of the loop to be adjusted to correspond with a user's head size.

In some embodiments, the end portion of the lower portion is telescopically attached to an end of the upper portion, in a telescoping arrangement, for example as described earlier. The position tabs are preferably attached to the upper portion, e.g. to an upper portion side of the flexible joints.

In some embodiments, at least one positioning tab is connected to at least one of the upper portion and the lower portion. In some embodiments the headgear members which are connected together to form a continuous loop may be connectable or disconnectable from each other so as to provide for locations where the continuous loop may be opened. This may be useful when the headgear needs to be removed from a user without disturbing other devices which may be attached to the user.

In some embodiments the continuous loop may be connectable and disconnectable between the headgear member and the interface (i.e., by connection arrangement 20), and/or between adjacent headgear members. If the headgear is telescoping as described above the sliding member 22 may be disconnectable from the headband 11 to break the continuous loop.

In some embodiments the headgear may comprises a channel 32 to allow for the passage of devices, or accessories (e.g. arms of a spectacle) from one side of the headgear to the other. The channel 32 may be located or form part of one or more of: the flexible joint 13, or the positioning tab 14 or positioning member 26, or the at least one headgear members, or the engagement portion 33.

In some embodiments the channel may form a channel passageway between a surface of the flexible joint, or the at least one headgear members, or the at least one positioning tab, or positioning member, or the engagement portion 33 and a patient's head. The channel 32 may extend from a front side of the headgear to a rear side of the headgear. Optionally, the channel 32 may extend from a front side of the flexible joint 13, or the positioning tab 14 or positioning member 26, or the at least one headgear members, or the engagement portion 33 to a rear side of the flexible joint 13, or the positioning tab 14 or positioning member 26, or the at least one headgear members, or the engagement portion 33.

As shown in Figures 11-11C, the channel may be provided by a substantially U-shaped portion. Optionally, the headgear may comprise engagement portions located on one or both sides of the channel 32 to rest on the users face in use. In some embodiments the channel may be provided as part of the engagement portions 33.

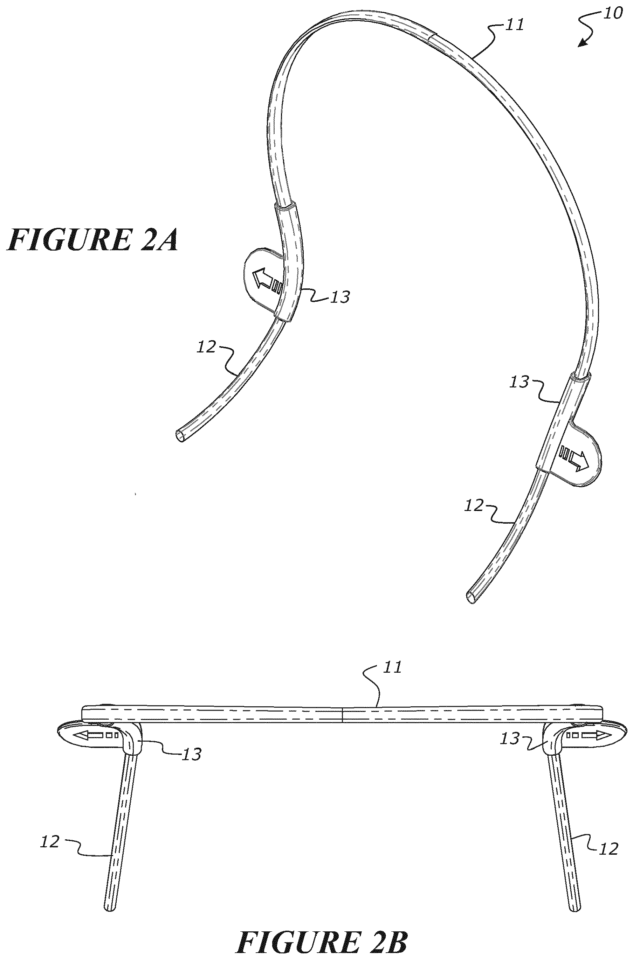

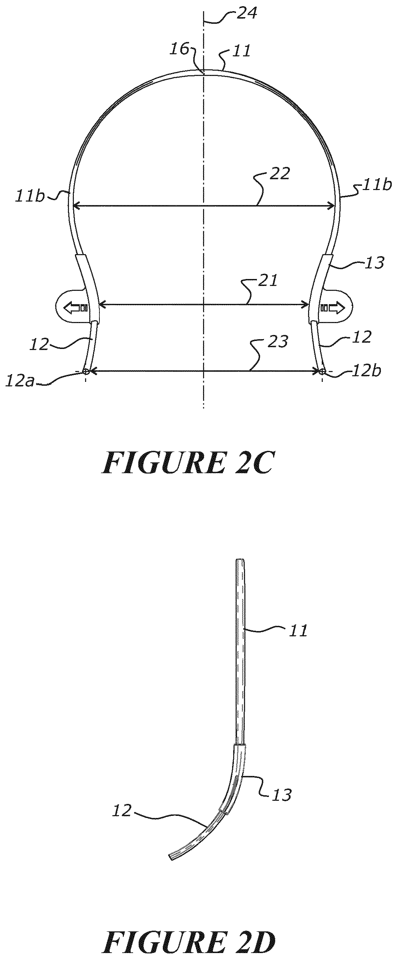

With reference to the Figures and particularly Figures 2D and 3D, the headband 11 is curved and predominantly lies in a plane, and the arms 12 each extend predominantly in a plane that is transverse to the plane of the headband. In the illustrated embodiments, each arm is curved to predominantly lie in a plane that is transverse to the plane of the headband.

In some embodiments, the flexible joints may provide for each arm to lie in a plane that is transverse to the plane of the headband, and/or the arms 12 may be curved predominantly lie in a plane that is transverse to the plane of the headband.

With reference to Figure 2C and 3C, in some embodiments, when the headgear is in use, e.g. positioned on a user's head to hold the interface 1 in place, and when viewed from a front (e.g. looking at the patient's face), the headgear forms a loop together with the interface 1, and wherein the loop has a narrow portion 21 between a top 11c of the headgear 10 and a bottom 12a, 12b of the headgear.

In some embodiments, the headgear 10 has a first lateral distance 21 between portions of the headgear that engage the user's head (e.g. the flexible joints 13 may contact the head and therefore provide or form engagement portions), a second lateral distance 22 between left and right side portions 11a, 11b of the headband 11 intermediate the engagement portions 13 and a sagittal plane position 11c of the headband, and a third lateral distance 23 between ends or end portions 12a, 12b of the headgear attached to or attachable to the patient interface 1. A lateral distance is across and perpendicular to the sagittal plane. In Figure 2C the sagittal plane is identified by reference numeral 24.

In some embodiments, the first lateral distance 21 is less than the second 22 and third 23 lateral distances. In some embodiments, the second lateral distance 22 is greater than the third lateral distance 23. In use, the user's head is clamped between the engagement portions located at the smallest lateral distance of the headgear. The engagement portions are biased against the user's head by the headband 11, as described earlier.

In some embodiments, this arrangement provides for a contact with the user's head at the engagement portions. A portion of the headgear between the engagement portions and the interface 1 may not contact or may have minimal contact with the head or face of the user. For example, the arms 12 may not bear against the user's face, or may make minimal contact with the user's face. The arms 12 may support side portions of the interface 1, e.g. side arms 4, away from the user's face, or support the interface or to make minimal contact with the user's face.

As described previously, in some embodiments, arm 12 of the headgear may be resilient, i.e. formed from a resilient material.

In some embodiments, the arms may be stiff to resist significant bending under normal operating conditions.

In some embodiments, the arms may be curved in a side view, with at least one centre of curvature forward of the side arm, for example as shown in Figures 2D and 3D. With ends of the headband located at sides of the patient's head, the curvature of the arms 12 provides for a correct positioning of the patient interface 1 on the face of the user and/or may assist to reduce contact between the interface and the user's face. Correct positioning of the interface may also be provided by flexible joints described above.

In some embodiments, the headgear may comprise a pair of flexible joints to attach the headgear to the patient interface, or the patient interface may comprise a pair of flexible joints to connect to the headgear. For example, the arms 12 may be connected to the patient interface by the pair of flexible joints. The flexible joints connecting the headgear to the interface may be as described earlier. For example, in some embodiments the arms 12 may be integrally formed with or are part of the patient interface, and the headband 11 may be connected via the flexible joints 13 to the patient interface side arms.

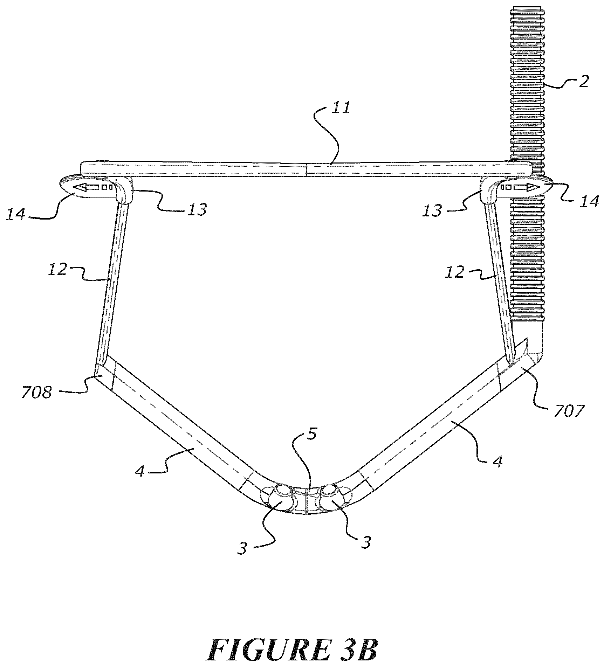

In some embodiments, one or both arms 12 may be integrally formed with a part of the patient interface, for example the 'plug' 708 and the 'conduit connector' 707 of the interface described in US provisional application 62/399893, as shown in Figures 3A and 3B of this present application.

In some embodiments, the arms 12 may be integrally formed with the headband in a single continuous member.

In some embodiments, the headgear may comprise two pairs of flexible joints 13, one pair connecting between the headband 11 and the arms 12, and one pair connecting between the arms 12 and the patient interface 1.

In some embodiments, one or each arm 12 may be attached to the patient interface by ball and socket joint. As described above, in some embodiments, the arms may be resilient. Alternatively, in some embodiments the arms 12 may be formed from soft flexible material. For example, the arms 12 may be integrally formed with the flexible joints 13.

An integrally formed flexible joint and arm member may comprise a flexible section forming the flexible joint, e.g. provided by a hollow section, and a less flexible or more rigid section forming the arm 12, e.g. provided by a solid cross section.

The cannula or interface can be made of flexible material so that the side arms 4 of the cannula or interface can flex relative to the prongs. This flexibility provides additional adjustability of the headgear relative to the prongs since the cannula or interface side arms can move relative to the prongs.

In some embodiments, the resilient headband 11, flexible joints 13 and/or resilient arms 12 may be or comprise conduits. For example, a resilient headband conduit may simultaneously secure the headgear to the patient's face and head while also providing a flow path to supply the patient interface with incoming gases. Conduits of the arm 12, flexible joint 13 and headband may connected together to be in fluid communication. For example, an end of the arm and an end of the headband may be received in the flexible joint.

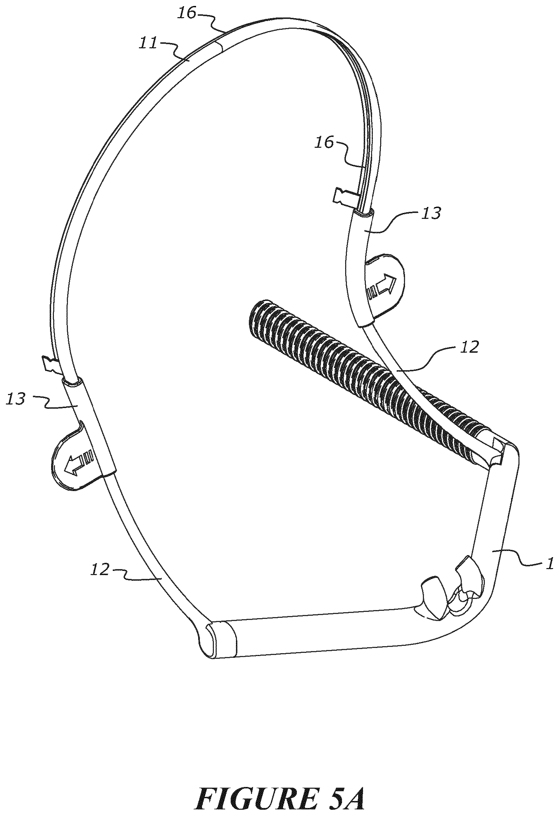

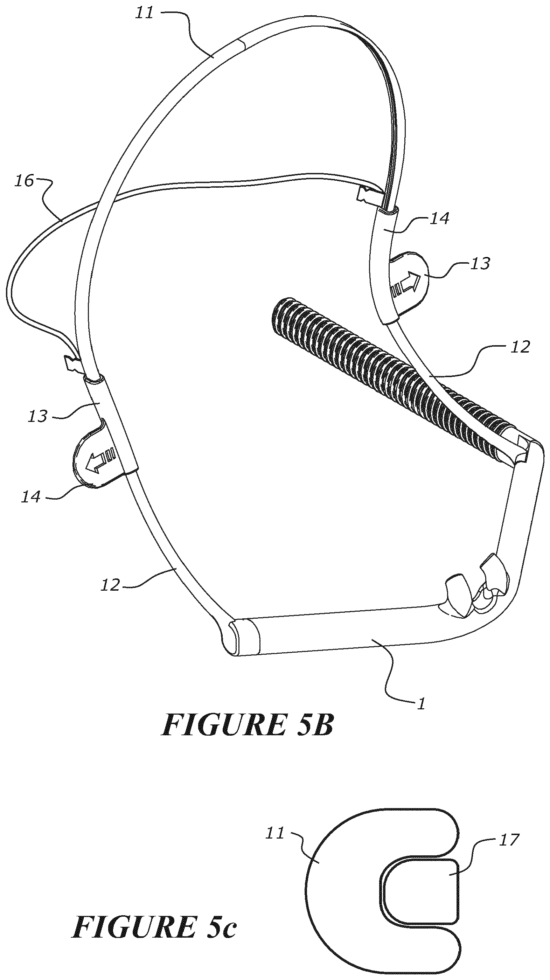

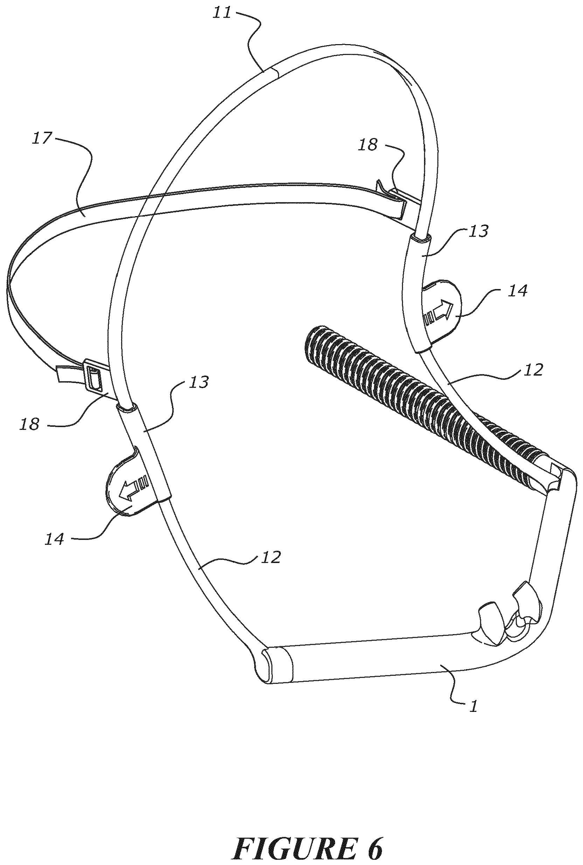

With reference to Figures 5A, 5B and 6, in some embodiments a second headband 16 may additionally be provided to enhance securement to the patient's head. The second headband 16 may be engageable with a rear part of a user's head, and optionally the second headband may engage with a portion of the user's head rearward of a crown of a user's head. The second headband 16 may be resilient, flexible with a reduced modulus of elasticity compared to the headband 11, or may be an elasticated strap or an elastic cord. The second headband may be an elastic strap or band. The elastic strap or band may be elastic, and/or stretchable and/or elongatable. The second headband may be detachable from the remaining headgear components. The second headband may attach or releasably attach to the resilient headband 11 or to the flexible joints 13. The flexible joints 13 may have a 'Y' shape to accommodate both the first headband 11 and the second headband 16.

In some embodiments for example as shown in Figure 5C the headband 11 may comprise a groove or cavity to store the second headband when the second headband is not required during use.

In some embodiments the groove or cavity may be a scallop, or valley and may be C-shaped. In a storage or non-use configuration the second headband, for example an elastic cord is retained in the groove or channel in an outer surface of the resilient headband 11, as presented in Figure 5A. The second headband may be removed from the groove or channel for use, as shown in Figure 5B. The second headband 16 may alternatively be retained by retention clips provided on the resilient headband 11.

In some embodiments the second headband 16 may be optionally provided to the headgear. The second headband may be optionally secured to the headband or arms or flexible joints by a connection feature to allow the second headband to be connected to the headband. Such a connection feature may comprise clips, connectors, grooves, or buckles for example.

Figure 6 illustrates a second headband 17 which is an elastic band which is optionally attachable to the headgear by connectors 18. The strap may be provided with a connector half and the first headband, flexible joint or arm may be provided with a corresponding connector half.

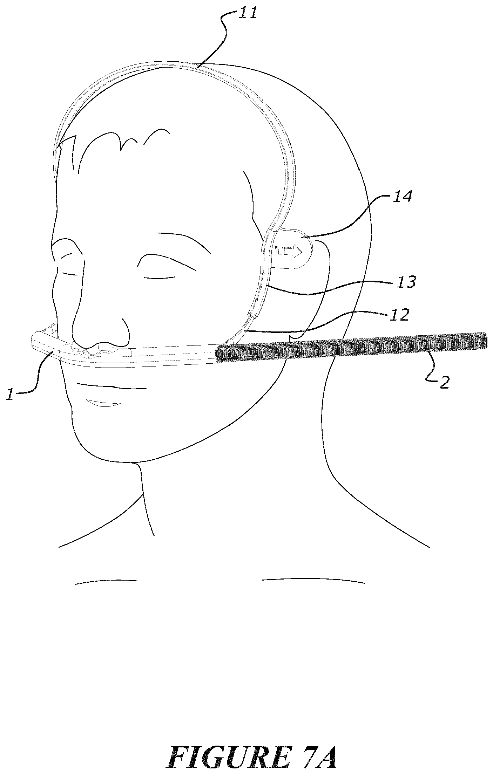

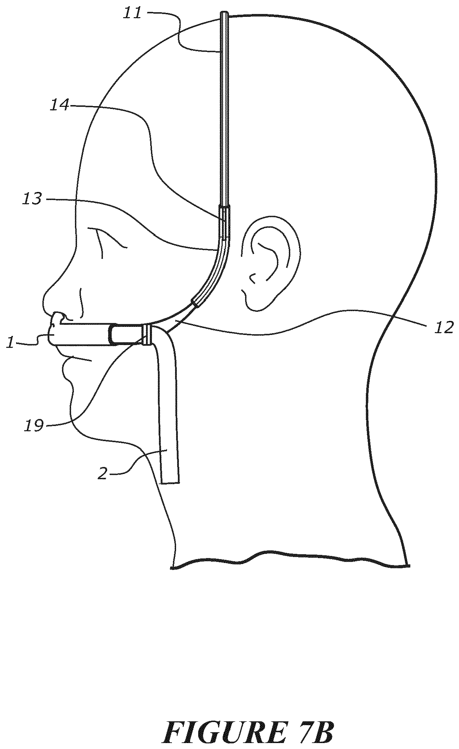

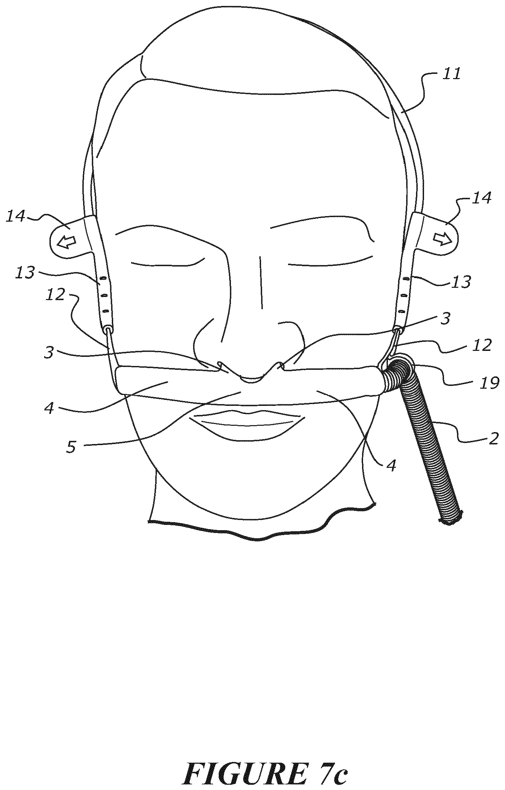

Figures 7A to 7C illustrate a headgear together with an interface that is similar to the headgear and interface shown in Figures 3A to 3D. In Figures 7A to 7C, the arms 12 of the headgear comprise a larger vertical dimension relative to the arms 12 of the embodiment shown in Figures 3A to 3D. In Figures 7A to 7C, the arms may contact the user's face, as shown in Figure 7C, to assist with supporting and maintaining a correct position of the interface on the user's face. As shown in Figures 7B and 7C, the headgear may have a band or other feature 19 to secure a breathing conduit to the arm 12.

Figures 11-11A illustrate a headgear connectable with an interface. The headgear 10 may comprise a headband comprising positioning members 26.

In some embodiments the positioning members 26 may act as flexible joints 13.

In some embodiments, the positioning members 26 may be used to fit the headgear to a user by a user pulling or applying force to the positioning members 26. This allows for extension of the profile of the headgear in a coronal plane of a user. The embodiment of Figures 11-11A also includes a channel 32 providing for a channel pathway between the user and the headgear 10.

Figures 13A-13C illustrate various headgears connectable with an interface. The headgear 10 comprises a pair of sliding members 22 which are configured to telescopically slide relative to the headgear member 11. The sliding members 22 comprise a connection arrangement 20 for connecting the headgear to an associated interface. The sliding members 22 of Figures 13A may have a flexible joint 34 to allow for movement between the sliding member 22 and the connecting arrangement 20. The flexible joint 34 may be a more flexible material than the remaining sliding member and/or connection arrangement, for example the flexible joint 34 may be made of a more flexible material (relative to the sliding member 22 and/or connection arrangement 20) and/or have a localised decrease in cross-sectional area (relative to the sliding member 22 and/or connection arrangement 20). The flexible joint 34 may be a living hinge.

In various embodiments, the sliding members 22 comprise positioning tabs or positioning members as described elsewhere in the present specification.

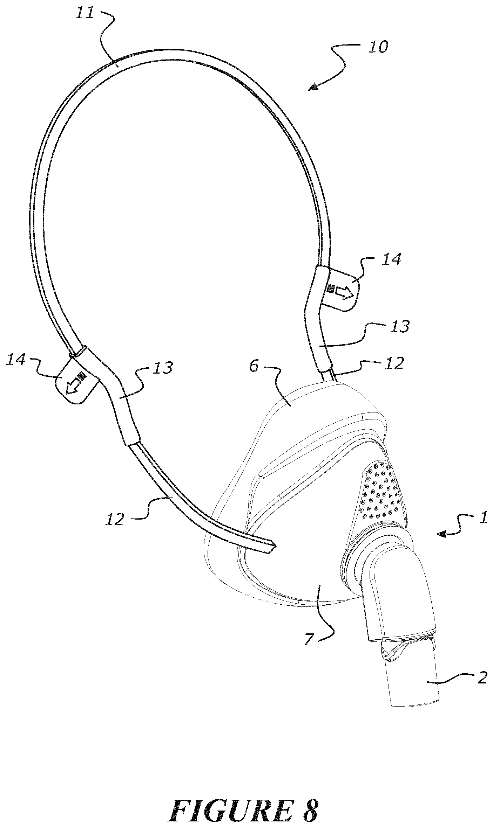

The various embodiments for a headgear described above are illustrated in Figures 1 and 3A to 7C together with a nasal cannula. However, a headgear according to described embodiments may be used together with other types of patient interfaces. For example, in Figure 8, a headgear according to embodiments described herein is shown together with a full face mask that covers the user's mouth and nose. The face mask comprises a seal 6 for contacting a user's face, and a body 7. The side arms 12 of the headgear may be connected to the body 7, by connectors, or may be formed with the body.

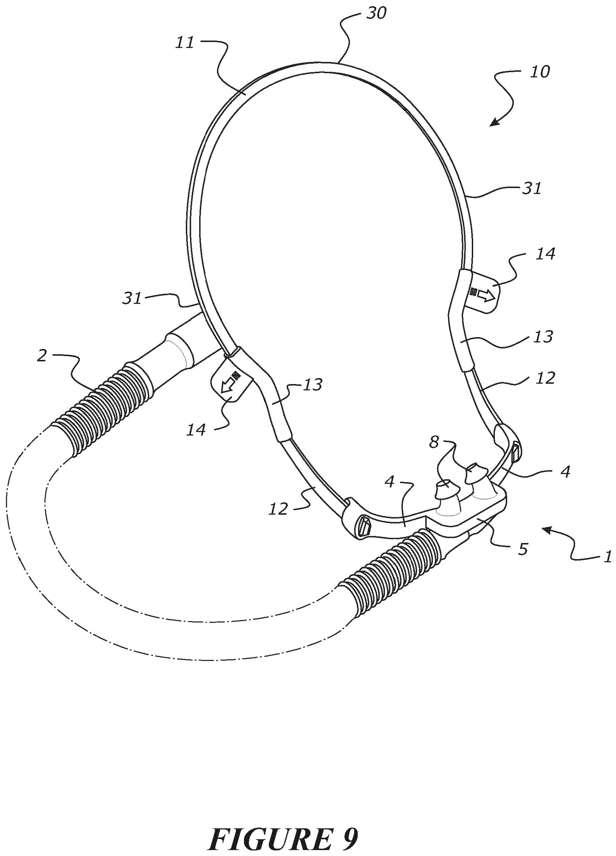

Figure 9 shows a headgear according to embodiments described herein together with a patient interface comprising nasal pillows to engage the nares of a user. Other under-nose interfaces may be used, for example interfaces that seal against or over a base of the user's nose.

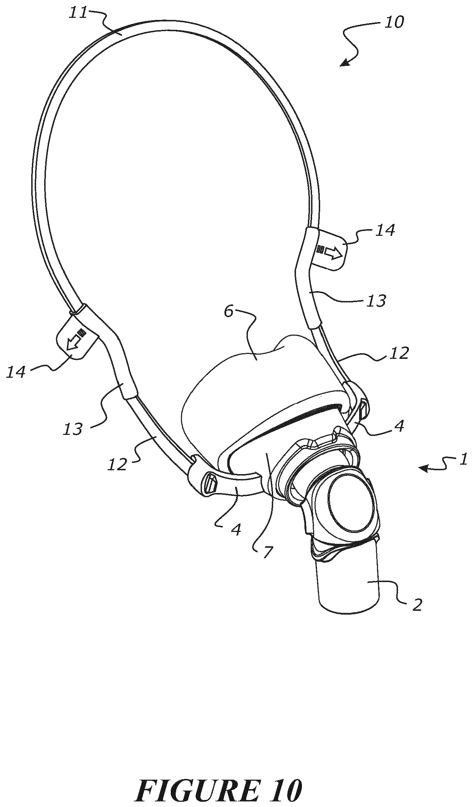

Figure 10 shows a headgear according to embodiments described herein together with a nasal interface. The nasal interface comprises a body 7 and a nasal seal 6 for sealing over the user's nose.

The headgear may also be provided as part of an assembly with a patient interface (for example as described above). The patient interface may be a nasal cannula, a full face mask, a nasal pillow mask, a nasal mask, an endotracheal tube, or a breath collector/sampler for sampling gases.

In some embodiments the interface may be a sealing or a non-sealing interface.

In some embodiments a method is provided for donning a headgear to a user's head. The method comprises applying a lateral force to each side or end of a resilient headband 11 against the inwards bias provided by the resilient headband to deform ends of the headband outwards. The method then comprises positioning the headband over the top of a user's head, and releasing the lateral force from each side or end of the resilient headband so that the bias of the headband forces engagement portions (e.g. flexible joints 13) of the headgear against sides of the user's head to hold the headgear to the user's head.

In some embodiments, the relative position between the headband and the patient interface is adjusted by flexing the flexible joints 13 for correct placement of the patient interface on user's nose, mouth or nose and mouth. The headband may be positioned over the top of a user's head, and/or the engagement portions may be located:
a) in proximity to the temporal bone of the user so that the engagement portions occupy an inward contour in front of the user's ear and above the zygomatic bone or arch when viewed in side profile, or
b) forward of the ear in proximity to the zygomatic bone when viewed in side profile.

In some embodiments, in the method of donning the headgear, the method includes gripping and pulling the pair of positioning tabs 14 to apply the lateral force to each side or end of a resilient headband of the headgear against the bias provided by the resilient headband to deform ends of the headband outwards. The outward force is then released to position the band correctly in place. The method may further comprise adjusting the position of the headband 11 or arm 12 to the flexible joint 13 for correct placement of the patient interface on user's nose, mouth or nose and mouth.

The method may further comprise applying the headband 11 to the head of the user and without applying a secondary headband or strap around the back of the user's head. The headband may be applied to the user's head with the user's head resting on a support (such as a pillow) and without lifting the user's head from the support, and/or the headband may be applied to the user's head without moving the user's head.

When a patient is under anaesthesia or is sedated, quick removal and application of a patient interface may take priority over patient comfort, for example due to an emergency. Further, comfort is less important in short term medical procedures compared to use of a respiratory interface during sleep, for example in treatment of OSA overnight.

Embodiments described herein may allow for rapid application and removal of the patient interface without disturbing the patient's hairnet or the position of the patient's head. The headgear configuration may disperse weight of the interface and tube 2 over the top of the user's head.

In some embodiments, the medical professional may adjust the angle of the headband relative to the arms 12 to an optimal position to best disperse the weight of the patient interface and the tubing. The headband may be tilted forward or backward to provide additional resistance to forces pulling away from the face. Some embodiments may provide for a large range of head sizes, for example by variation in fit provided by the flexible joints 13 and/or adjustment in longitudinal position between headgear members. A further advantage is that the patient interface is held in a stable position on the patient's face, due to being resiliently supported from each side of the user's head.

The foregoing description of the invention includes preferred forms thereof. Modifications may be made thereto without departing from the scope of the invention as defined by the accompanying claims.

## Claims

1. A headgear (10) for a patient interface (1) comprising:
at least one headgear member, wherein the at least one headgear member is a resilient headband (11);
a pair of positioning tabs (14) or positioning members (26) connected to, or forming part of, at least one said headgear member, each positioning tab (14) or positioning member (26) adjacent an end or end portion of the resilient headband (11) to be located at a side of the user's head in use; and
a pair of flexible joints (13, 34) adapted to connect said the at least one headgear member to the patient interface (1) and/or a pair of flexible joints (13, 34) connecting one of the headgear members to an adjacent headgear member,
each flexible joint (13, 34) allowing free relative movement between the at least one headgear member and the patient interface (1) or between the adjacent headgear members,
wherein the resilient headband (11) is shaped to fit a user's head with each end of the resilient headband (11) located to and biased against a respective side of the user's head, and
wherein the resilient headband (11) is configured such that in an undeflected condition the distance between the ends of the resilient headband (11) is less than a width of a user's head so that, in use, deflection of the resilient headband (11) to fit the user's head biases the ends of the resilient headband (11) against sides of the user's head.

2. The headgear (10) of claim 1, wherein each of the positioning tabs (14) or positioning members (26) is provided by or comprises a substantially u-shaped portion.

3. The headgear (10) of claim 1 or claim 2, wherein each of the positioning tabs (14) or positioning members (26) comprises:
a first portion (27) extending outward from the at least one headgear member in use, and
a second portion (28) extending toward the at least one headgear member in use.

4. The headgear (10) of claim 3, wherein each of the positioning tabs (14) or positioning members (26) comprises an intermediate portion (29) located intermediate the first portion (27) and the second portion (28), the intermediate portion (29) extending substantially parallel to the at least one headgear member in use.

5. The headgear (10) of any one of claims 1 to 3, wherein the positioning tabs (14) or positioning members (26) are integrally formed with the at least one headgear member.

6. The headgear (10) of any one of the preceding claims, comprising a pair of engagement portions (33), each engagement portion (33) configured to engage a side of the user's head in use.

7. The headgear (10) of claim 6, wherein the pair of flexible joints (13, 34) and/or the at least one headgear member provide or comprise the engagement portions (33).

8. The headgear (10) of claim 6 or claim 7, wherein the engagement portions (33) comprise a textured surface, optionally wherein the textured surface comprises one or more of: a knurled portion, a waved surface, a ribbed surface, a roughened surface, or may comprise micro or nano projections.

9. The headgear (10) of any one of claims 1 to 8, wherein the flexible joint (13, 34), or each of the positioning tabs (14), or the at least one headgear member comprises at least one channel (32).

10. The headgear (10) of claim 9, wherein the channel (32) extends from a front side of the flexible joint (13, 34), or the at least one headgear member to a rear side of the flexible joint (13, 34), or the at least one headgear member, or the or an engagement portion (33).

11. The headgear (10) of any preceding claim, wherein the positioning tabs (14) or positioning members (26) are integrally formed with the flexible joints (13, 34).

12. The headgear (10) of any one of claims 1 to 11,
wherein the headgear (10) further comprises a pair of arms (12), each arm (12) attached to the resilient headband (11), each arm (12) being adapted to connect to the patient interface (1), and
wherein the headgear (10) has a first lateral distance (21) between the flexible joints (13, 34), a second lateral distance (22) between left and right side portions of the resilient headband (11), and a third lateral distance (23) between ends or end portions of the arms (12) attached to or attachable to the patient interface (1),
wherein each of the first lateral distance (21), the second lateral distance (22) and the third lateral distance (23) is across and perpendicular to a sagittal plane position (24) of the resilient headband (11), and
wherein the first lateral distance (21) is less than the second lateral distance (22) and the third lateral distance (23).

13. The headgear (10) of any one of claims 1 to 12,
wherein the modulus of elasticity of the at least one of the headgear members is at least about 0.5 GPa, or at least about 1GPa, or at least about 1.5GPa, or at least about 1.8GPa, or at least about 2GPa, or at least about 3 GPa.

14. The headgear of any one of the preceding claims, wherein the resilient headband (11) has a central portion (30), and two end portions (31), wherein the central portion is stiffer and/or has a larger cross-sectional area than the end portions.

15. A patient interface assembly comprising:
a patient interface (1),
a headgear (10), wherein the headgear (10) is the headgear (10) of any one of claims 1 to 14, the headgear (10) configured to, in use, locate the patient interface (1) upon a patient's face.

## Patentansprüche

1. Kopfausrüstung (10) für eine Patientenschnittstelle (1), umfassend:
mindestens ein Kopfausrüstungselement, wobei das mindestens eine Kopfausrüstungselement ein elastisches Kopfband (11) ist;
ein Paar Positionierlaschen (14) oder Positionierelemente (26), die mit mindestens einem Kopfausrüstungselement verbunden sind oder einen Formierabschnitt davon bilden, wobei jede Positionierlasche (14) oder jedes Positionierelement (26) an ein Ende oder einen Endabschnitt des elastischen Kopfbands (11) angrenzt, das im Gebrauch an einer Seite des Kopfes des Benutzers anzuordnen ist; und
ein Paar flexibler Gelenke (13, 34), das dazu ausgebildet ist, das mindestens eine Kopfausrüstungselement mit der Patientenschnittstelle (1) zu verbinden, und/oder ein Paar flexibler Gelenke (13, 34), das eines der Kopfausrüstungselemente mit einem angrenzenden Kopfausrüstungselement verbindet,
wobei jedes flexible Gelenk (13, 34) eine freie relative Bewegung zwischen dem mindestens einen Kopfausrüstungselement und der Patientenschnittstelle (1) oder zwischen den angrenzenden Kopfausrüstungselementen ermöglicht,
wobei das elastische Kopfband (11) so geformt ist, dass es auf den Kopf eines Benutzers passt, wobei jedes Ende des elastischen Kopfbandes (11) an einer jeweiligen Seite des Kopfes des Benutzers angeordnet und gegen diese vorgespannt ist, und
wobei das elastische Kopfband (11) konfiguriert ist, um in einem nicht gebogenen Zustand einen Abstand zwischen den Enden des elastischen Kopfbands (11) aufzuweisen, der kleiner ist als die Kopfbreite eines Benutzers, sodass bei der Verwendung durch das Biegen des elastischen Kopfbands (11) zur Anpassung an den Kopf des Benutzers die Enden des elastischen Kopfbands (11) gegen die Seiten des Kopfes des Benutzers vorgespannt werden.

2. Kopfausrüstung (10) nach Anspruch 1, wobei jede der Positionierungslaschen (14) oder jedes der Positionierungselemente (26) durch einen im Wesentlichen u-förmigen Abschnitt bereitgestellt wird oder einen im Wesentlichen u-förmigen Abschnitt umfasst.

3. Kopfausrüstung (10) nach Anspruch 1 oder Anspruch 2, wobei jede der Positionierungslaschen (14) oder jedes der Positionierungselemente (26) umfasst:
einen ersten Abschnitt (27), der sich im Gebrauch von dem mindestens einen Kopfausrüstungselement nach außen erstreckt, und
einen zweiten Abschnitt (28), der sich im Gebrauch in Richtung des mindestens einen Kopfausrüstungselements erstreckt.

4. Kopfausrüstung (10) nach Anspruch 3, wobei jede der Positionierungslaschen (14) oder jedes der Positionierungselemente (26) einen Zwischenabschnitt (29) umfasst, der zwischen dem ersten Abschnitt (27) und dem zweiten Abschnitt (28) angeordnet ist, wobei sich der Zwischenabschnitt (29) im Gebrauch im Wesentlichen parallel zu dem mindestens einen Kopfausrüstungselement erstreckt.

5. Kopfausrüstung (10) nach einem der Ansprüche 1 bis 3, wobei die Positionierungslaschen (14) oder Positionierungselemente (26) einstückig mit dem mindestens einen Kopfausrüstungselement ausgebildet sind.

6. Kopfausrüstung (10) nach einem der vorstehenden Ansprüche, umfassend ein Paar Eingriffsabschnitte (33), wobei jeder Eingriffsabschnitt (33) konfiguriert ist, um im Gebrauch mit einer Seite des Kopfes des Benutzers in Eingriff zu kommen.

7. Kopfausrüstung (10) nach Anspruch 6, wobei das Paar flexibler Gelenke (13, 34) und/oder das mindestens eine Kopfausrüstungselement die Eingriffsabschnitte (33) bereitstellen oder umfassen.

8. Kopfausrüstung (10) nach Anspruch 6 oder Anspruch 7, wobei die Eingriffsabschnitte (33) eine strukturierte Oberfläche umfassen, wobei optional die strukturierte Oberfläche eines oder mehrere umfasst von: einem gerändelten Abschnitt, einer gewellten Oberfläche, einer gerippten Oberfläche, einer aufgerauten Oberfläche, oder Mikro- oder Nanovorsprünge umfassen kann.

9. Kopfausrüstung (10) nach einem der Ansprüche 1 bis 8, wobei das flexible Gelenk (13, 34) oder jede der Positionierungslaschen (14) oder das mindestens eine Kopfausrüstungselement mindestens einen Kanal (32) umfasst.

10. Kopfausrüstung (10) nach Anspruch 9, wobei sich der Kanal (32) von einer Vorderseite des flexiblen Gelenks (13, 34) oder des mindestens einen Kopfausrüstungselements zu einer Rückseite des flexiblen Gelenks (13, 34) oder des mindestens einen Kopfausrüstungselements oder des oder eines Eingriffsabschnitts (33) erstreckt.

11. Kopfausrüstung (10) nach einem der vorstehenden Ansprüche, wobei die Positionierungslaschen (14) oder Positionierungselemente (26) einstückig mit den flexiblen Gelenken (13, 34) ausgebildet sind.

12. Kopfausrüstung (10) nach einem der Ansprüche 1 bis 11,
wobei die Kopfausrüstung (10) ferner ein Paar Arme (12) umfasst, wobei jeder Arm (12) an dem elastischen Kopfband (11) befestigt ist, wobei jeder Arm (12) dazu ausgebildet ist, mit der Patientenschnittstelle (1) verbunden zu werden, und
wobei die Kopfausrüstung (10) einen ersten lateralen Abstand (21) zwischen den flexiblen Gelenken (13, 34), einen zweiten lateralen Abstand (22) zwischen linken und rechten Seitenabschnitten des elastischen Kopfbands (11) und einen dritten lateralen Abstand (23) zwischen Enden oder Endabschnitten der Arme (12), die an der Patientenschnittstelle (1) angebracht oder anbringbar sind, aufweist,
wobei jeder von dem ersten lateralen Abstand (21), dem zweiten lateralen Abstand (22) und dem dritten lateralen Abstand (23) quer und senkrecht zu einer Sagittalebenenposition (24) des elastischen Kopfbandes (11) verläuft, und
wobei der erste laterale Abstand (21) kleiner als der zweite laterale Abstand (22) und der dritte laterale Abstand (23) ist.

13. Kopfausrüstung (10) nach einem der Ansprüche 1 bis 12,
wobei der Elastizitätsmodul des mindestens einen der Kopfausrüstungselemente mindestens etwa 0,5 GPa, oder mindestens etwa 1 GPa, oder mindestens etwa 1,5 GPa, oder mindestens etwa 1,8 GPa, oder mindestens etwa 2 GPa, oder mindestens etwa 3 GPa beträgt.

14. Kopfausrüstung nach einem der vorstehenden Ansprüche, wobei das elastische Kopfband (11) einen Mittelabschnitt (30) und zwei Endabschnitte (31) aufweist, wobei der Mittelabschnitt steifer ist und/oder eine größere Querschnittsfläche als die Endabschnitte aufweist.

15. Patientenschnittstellenanordnung, umfassend:
eine Patientenschnittstelle (1),
eine Kopfausrüstung (10), wobei die Kopfausrüstung (10) die Kopfausrüstung (10) nach einem der Ansprüche 1 bis 14 ist, wobei die Kopfausrüstung (10) konfiguriert ist, um im Gebrauch die Patientenschnittstelle (1) auf dem Gesicht eines Patienten zu positionieren.

## Revendications

1. Équipement de tête (10) destiné à une interface patient (1), comprenant :
au moins un élément d'équipement de tête, dans lequel l'au moins un élément d'équipement de tête est un serre-tête élastique (11) ;
une paire de languettes de positionnement (14) ou d'éléments de positionnement (26) reliés à, ou faisant partie de, au moins un dit élément d'équipement de tête, chaque languette de positionnement (14) ou élément de positionnement (26) étant adjacent à une extrémité ou à une partie d'extrémité du serre-tête élastique (11) à placer au niveau d'un côté de la tête de l'utilisateur lors de l'utilisation ; et
une paire d'articulations flexibles (13, 34) adaptées pour relier ledit au moins un élément d'équipement de tête à l'interface patient (1) et/ou une paire d'articulations flexibles (13, 34) reliant l'un des éléments d'équipement de tête à un élément d'équipement de tête adjacent,
chaque articulation flexible (13, 34) permettant un mouvement relatif libre entre l'au moins un élément d'équipement de tête et l'interface patient (1) ou entre les éléments d'équipement de tête adjacents,
dans lequel le serre-tête élastique (11) est formé pour s'ajuster à la tête d'un utilisateur, chaque extrémité du serre-tête élastique (11) étant située sur un côté respectif de la tête de l'utilisateur et sollicitée contre celui-ci, et
dans lequel le serre-tête élastique (11) est conçu de telle sorte que dans un état non défléchi la distance entre les extrémités du serre-tête élastique (11) est inférieure à une largeur de la tête d'un utilisateur de sorte que, lors de l'utilisation, la déviation du serre-tête élastique (11) pour l'ajuster à la tête de l'utilisateur sollicite les extrémités du serre-tête résilient (11) contre les côtés de la tête de l'utilisateur.

2. Équipement de tête (10) selon la revendication 1, dans lequel chacune des languettes de positionnement (14) ou des éléments de positionnement (26) est fournie par ou comprend une partie sensiblement en forme de U.

3. Équipement de tête (10) selon la revendication 1 ou la revendication 2, dans lequel chacune des languettes de positionnement (14) ou des éléments de positionnement (26) comprend :
une première partie (27) s'étendant vers l'extérieur à partir de l'au moins un élément d'équipement de tête en cours d'utilisation, et
une seconde partie (28) s'étendant vers l'au moins un élément d'équipement de tête en cours d'utilisation.

4. Équipement de tête (10) selon la revendication 3, dans lequel chacun des languettes de positionnement (14) ou des éléments de positionnement (26) comprend une partie intermédiaire (29) située entre la première partie (27) et la seconde partie (28), la partie intermédiaire (29) s'étendant sensiblement parallèlement à l'au moins un élément d'équipement de tête en cours d'utilisation.

5. Équipement de tête (10) selon l'une quelconque des revendications 1 à 3, dans lequel les languettes de positionnement (14) ou les éléments de positionnement (26) sont formés d'un seul tenant avec l'au moins un élément d'équipement de tête.

6. Équipement de tête (10) selon l'une quelconque des revendications précédentes, comprenant une paire de parties de mise en prise (33), chaque partie de mise en prise (33) étant conçue pour venir en prise avec un côté de la tête de l'utilisateur lors de l'utilisation.

7. Équipement de tête (10) selon la revendication 6, dans lequel la paire d'articulations flexibles (13, 34) et/ou l'au moins un élément d'équipement de tête fournissent ou comprennent les parties de mise en prise (33).

8. Équipement de tête (10) selon la revendication 6 ou la revendication 7, dans lequel les parties de mise en prise (33) comprennent une surface texturée, éventuellement dans lequel la surface texturée comprend un ou plusieurs parmi : une partie moletée, une surface ondulée, une surface nervurée, une surface rugueuse, ou peut comprendre des micro ou nanosaillies.

9. Équipement de tête (10) selon l'une quelconque des revendications 1 à 8, dans lequel l'articulation flexible (13, 34), ou chacune des languettes de positionnement (14), ou l'au moins un élément d'équipement de tête comprend au moins un canal (32).

10. Équipement de tête (10) selon la revendication 9, dans lequel le canal (32) s'étend à partir d'un côté avant du joint flexible (13, 34), ou de l'au moins un élément d'équipement de tête vers un côté arrière du joint flexible (13, 34), ou de l'au moins un élément d'équipement de tête, ou de la ou d'une partie de mise en prise (33).

11. Équipement de tête (10) selon l'une quelconque revendication précédente, dans lequel les languettes de positionnement (14) ou les éléments de positionnement (26) sont formés d'un seul tenant avec les joints flexibles (13, 34).

12. Équipement de tête (10) selon l'une quelconque des revendications 1 à 11,
dans lequel l'équipement de tête (10) comprend en outre une paire de bras (12), chaque bras (12) étant attaché au serre-tête élastique (11), chaque bras (12) étant adapté pour se connecter à l'interface patient (1), et
dans lequel l'équipement de tête (10) a une première distance latérale (21) entre les articulations flexibles (13, 34), une deuxième distance latérale (22) entre les parties latérales gauche et droite du serre-tête élastique (11), et une troisième distance latérale (23) entre des extrémités ou des parties d'extrémité des bras (12) fixés à ou pouvant être fixés à l'interface patient (1),
dans lequel chacune de la première distance latérale (21), de la deuxième distance latérale (22) et de la troisième distance latérale (23) est transversale et perpendiculaire à une position de plan sagittal (24) du serre-tête élastique (11), et
dans lequel la première distance latérale (21) est inférieure à la deuxième distance latérale (22) et à la troisième distance latérale (23).

13. Équipement de tête (10) selon l'une quelconque des revendications 1 à 12.
dans lequel le module d'élasticité de l'au moins un des éléments d'équipement de tête est d'au moins environ 0,5 GPa, ou d'au moins environ 1 GPa, ou d'au moins environ 1,5 GPa, ou d'au moins environ 1,8 GPa, ou d'au moins environ 2 GPa, ou d'au moins environ 3 GPa.

14. Équipement de tête selon l'une quelconque des revendications précédentes, dans lequel le serre-tête élastique (11) a une partie centrale (30) et deux parties d'extrémité (31), dans lequel la partie centrale est plus rigide et/ou a une section transversale plus grande que les parties d'extrémité.

15. Ensemble d'interface patient comprenant :
une interface patient (1),
un équipement de tête (10), dans lequel l'équipement de tête (10) est l'équipement de tête (10) selon l'une quelconque des revendications 1 à 14, l'équipement de tête (10) étant conçu pour, en cours d'utilisation, localiser l'interface patient (1) sur le visage d'un patient.
